# EUROPEAN PATENT SPECIFICATION

(11) **EP 0 734 243 B1**
(45) Date of publication and mention of the grant of the patent: **28.06.2000**
(21) Application number: 95907222.4
(22) Date of filing: 14.12.1994
(51) Int. Cl.: A61F 13/58, A61F 13/62

(54) **DYNAMIC FITTING DIAPER**
WINDEL MIT EINER DYNAMISCHEN PASSUNG ZUM KÖRPER EINES TRÄGERS
COUCHE-CULOTTE A AJUSTEMENT DYNAMIQUE

(30) Priority: 16.12.1993 US 168615
(43) Date of publication of application: 02.10.1996
(73) Proprietor: KIMBERLY-CLARK WORLDWIDE, INC., Neenah, Wisconsin 54956 (US)
(72) Inventor: ROESSLER, Thomas Harold, Menasha, WI 54952 (US); VAN GOMPEL, Paul Theodore, Hortonville, WI 54944 (US); HUANG, Yung Hsiang, Appleton, WI 54914 (US); ZEHNER, Georgia Lynn, Larsen, WI 54947 (US)
(74) Representative: DIEHL GLAESER HILTL & PARTNER
(86) International application number: US9414529
(87) International publication number: WO9516425

(56) References cited:
- EP-A- 0 113 464
- EP-A- 0 532 034
- WO-A-93/24085
- FR-A- 2 624 353
- US-A- 4 066 081

## Description

### Field of the Invention

The present invention relates to fastening systems for disposable garments, such as caps, gowns, diapers, shoe covers, incontinence garments and the like. More particularly, the present invention relates to adhesive tape fastening systems and interlocking, mechanical-type fastening systems for disposable articles, such as gowns, diapers, incontinence garments and the like.

### Background of the Invention

Conventional disposable absorbent articles have typically employed adhesive fastening tapes for securing the article on a wearer. For example, see US-A-2714889 issued August 9, 1955, to U. Chambers and US-A-4050462 issued September 27, 1977, to L. Woon et al. Conventional adhesive tape fastening systems have employed adhesive tape tabs which include a non-adhesive section located at the distal free end of the tape tab. This adhesive-free region has typically referred to as a finger tab for facilitating the grasping of the end of the adhesive tape. For example, US-A-4055182 issued October 25, 1977, to R. Mack describes an end tab formed by folding the end region of the tab back onto itself. Other adhesive tape structures have included a finger tab formed by placing a separate piece of material at the terminal free end of the tape member. For example, see US-A-4726971 issued February 23, 1988, to P. Pape et al.; US-A-3616114 issued October 26, 1971, to T. Hamaguchi et al.; US-A-4801480 issued January 31, 1989, to V. Panza et al.

Other articles have included a fastening system which extends along substantially the entire length of an ear section of the article. Still other conventional fastening systems have employed tapered fastening tabs where the user's end is relatively wide at the longitudinally extending sides of the diaper, and is tapered to a more narrow width at its distal end. For example, see European Patent 0 233 704 B1 of H. Burkhard et al.

Conventional fastening systems, such as those described above, have not provided an adequate level of dynamic fit in combination with a neat tailored appearance and reliable securement. The conventional fastening systems have not provided a sufficient capability to move and adjust to accommodate the stresses and displacements caused by a moving wearer. As a result, the fastening systems have not provided desired levels of reliable securement and comfort.

### Brief Description of the Invention

Generally stated, the present invention provides a distinctive article having a front waistband portion, a rear waistband portion and an intermediate portion interconnecting the front and rear waistband portions. The article includes a backsheet layer, and a pair of side panels, each of which extends laterally at opposed lateral ends of at least one waistband portion of the article. Each of the side panels includes a terminal free end region which has a predetermined length dimension thereof. A stress beam section is connected to each of the side panels along the free end region, and the beam section provides for a relatively high Gurley stiffness value and has a length dimension which is at least a significant, substantial percentage of the length of the free end region of the side panel. A fastening tab is connected to each of the stress beam sections and is arranged to extend laterally from each of the side panels for securing together the front and rear waistband portions of the article during use thereof. In particular aspects of the invention, the fastening tab can have a base length which is not more than a selected, limited percentage of the length of the stress beam section.

In another aspect of the invention, an article has a front waistband portion, a rear waistband portion and an intermediate portion interconnecting said front and rear waistband portions. The article includes a backsheet layer, and a fastening means connected to opposed lateral ends of at least one waistband portion of said backsheet layer for securing said article on a wearer. The fastening means having a factory bond section, a user bond section, and a seam section which is located between said factory bond and user bond sections. The user bond section having a length dimension which is larger than a length dimension of said seam section.

Yet another aspect of the invention can provide a fastener article which comprises a tab substrate having a factory bond section, a user bond section, and a seam section which is located between said factory bond and user bond sections. The user bond section has a length dimension which is larger than a length dimension of said seam section.

In its various aspects, the distinctive fastening system of the present Invention can advantageously provide an improved combination of neat appearance and dynamic fit. The closure stresses can be more efficiently distributed along the length of the side panel sections of the article. In addition, the interconnected front and rear waistband sections of the article can more effectively move relative to each other while maintaining a secure fastening therebetween. The front and rear waistband portions of the article can more effectively shift and move to accommodate the stresses and displacements caused by the movements of a wearer. In particular aspects of the invention, the fastening system can provide for a more effective cooperation between the elastics at the waistband region of the article and the elastics at the leg openings of the article. As a result, the various aspects of the fastening system of the invention can provide improved securement with fewer pop-opens, and can also provide improved fit, greater comfort, reduced irritation and reduced red marking of the wearer's skin.

### Brief Description of the Drawings

The present invention will be more fully understood and further advantages will become apparent when reference is made to the following detailed description of the invention and the drawings, in which:
Fig. 1 representatively shows a partially cut-away plan view of a diaper article of the invention;
Fig. 2 representatively shows a plan view of a side panel and fastening tab assembly of the invention, wherein the length dimension of the stress beam section is less than the length of the free end region of the associated side panel member;
Fig. 3 representatively shows cross-sectional side view of the side panel and fastening tab assembly representatively shown in Fig. 2;

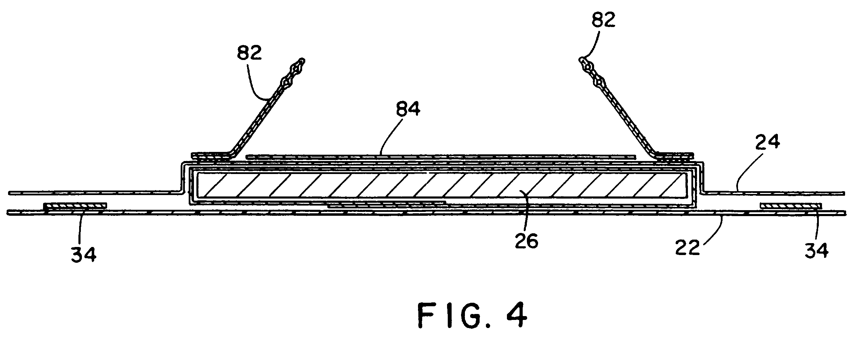

Fig. 4 representatively shows a cross-sectional view of an embodiment of the diaper article of the invention having a surge management layer and a pair of containment flaps;
Fig. 5 representatively shows a partially cut-away plan view of another embodiment of the diaper article of the invention having a non-rectangular, contoured side panel configuration;
Fig. 6 representatively shows a plan view of a side panel and fastening tab assembly of the invention, wherein the stress beam section is formed from a piece of material which is separate from the material employed to form the fastening tab and wherein the length dimension of the stress beam section is substantially equal to the length of the free end region of the associated side panel member;
Fig. 7 representatively shows cross-sectional side view of the side panel and fastening tab assembly representatively shown in Fig. 6; Some of the further additional Figures are not concerned with the inventive concept of the present invention but only illustrate certain further aspects. These Figures will be denominated "illustrative only" in the following.
Fig. 8 - illustrative only - representatively shows a plan view of a side panel and fastening tab assembly, wherein the stress beam section is formed from a piece of material which is separate and spaced away from the material employed to form the fastening tab;
Fig. 9 - illustrative only - representatively shows a cross-sectional side view of the side panel and fastening tab assembly representatively shown in Fig. 8;
Fig. 10 - illustrative only - representatively shows an aspect having a plurality of stress beam elements;
Fig. 11 - illustrative only - representatively shows the cross-sectional side view of the side panel and fastening tab assembly representatively shown in Fig. 10;
Fig. 12 - illustrative only - representatively shows an aspect having a plurality of stress beam elements constructed and arranged at one or more selected relative angles with respect to each other;
Fig. 13 - illustrative only - representatively shows an aspect having a plurality of stress beam elements wherein the stress beam elements can be constructed and arranged with different, selected lengths thereof;
Fig. 14 - illustrative only - representatively shows cross-sectional side view of the side panel and fastening tab assembly representatively shown in Fig. 13;
Fig. 15 representatively shows a fastener system having a primary stress beam section and a supplemental stress beam section;
Fig. 16 representatively shows another fastener system having a primary stress beam section and a supplemental stress beam section, wherein the supplemental stress beam section is spaced a discrete distance from a lateral edge of a waistband of a diaper article;
Fig. 17 representatively shows a fastener system having a stress beam section connected to a side panel that is integral with a backsheet layer of a diaper article;
Fig. 18 illustrative only representatively shows a fastener system without a stress beam and with a narrow fastener tab;
Fig. 18A illustrative only representatively shows a cross-sectional, side view of the fastener system of Fig. 18;
Fig. 19 illustrative only representatively shows another fastener system without a stress beam and with a medium-sized fastener tab;
Fig. 19A illustrative only representatively shows a cross-sectional, side view of the fastener system of Fig. 19;
Fig. 20 illustrative only representatively shows a fastener system with a stress beam section and a relatively narrower fastener tab;
Fig. 20A illustrative only representatively shows a cross-sectional, side view of the fastener system of Fig. 20;
Fig. 21 illustrative only representatively shows a fastener system having a large fastener tab which extends along substantially the entire length of a side panel member;
Fig. 21A illustrative only representatively shows a cross-sectional, side view of the fastener system of Fig. 21;
Fig. 22 shows a graph which representatively shows the comparative effects of tape tab size and stress beam size on the tensile load carried by the composite fastener system;
Fig. 23 illustrative only representatively shows a plan view of an adhesive-bearing, securing surface of a fastening tab having a user bond portion with a length dimension which is equal to or less than a length dimension of an intermediate section of the tab;
Fig. 23A illustrative only representatively shows a cross-sectional, side view of the test sample illustrated in Fig. 23.
Fig. 24 illustrative only representatively shows a plan view of an adhesive-bearing, securing surface of a fastening tab having a user band portion with a length dimension which is equal to a length dimension of an intermediate section of the tab;
Fig. 24A illustrative only representatively shows a cross-sectional, side view of the test sample illustrated in Fig. 24.
Fig. 25 representatively shows a plan view of an adhesive-bearing, securing surface of a fastening tab having a user bond portion with a length dimension which is equal to or greater than a length dimension of an intermediate section of the tab;
Fig. 25A representatively shows a cross-sectional, side view of the test sample illustrated in Fig. 25.
Fig. 26 illustrative only representatively shows a top view of a sample of a fastener tab which is prepared for shear testing; and
Fig. 26A illustrative only representatively shows a side view of the prepared test sample illustrated in Fig. 26.

### Detailed Description of the Invention

The various embodiments of the invention will be described in the context of a disposable absorbent article, such as a disposable diaper. It Is, however, readily apparent that the present invention could also be employed with other articles, such as caps, gowns, shoe covers, feminine care articles, incontinence garments and the like.

Typically, disposable articles are intended for limited use and are not intended to be laundered or otherwise cleaned for reuse. For example, a disposable diaper is discarded after it has become soiled by the wearer.

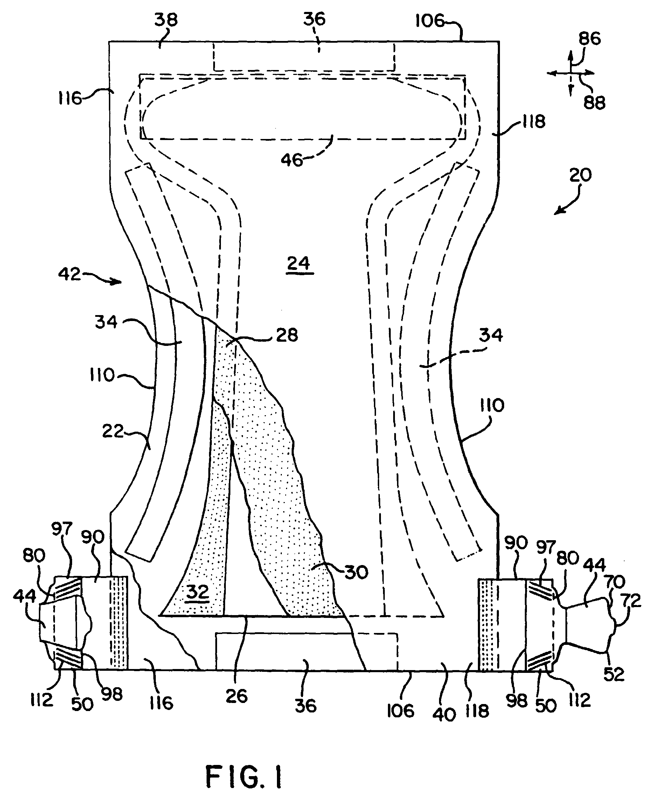

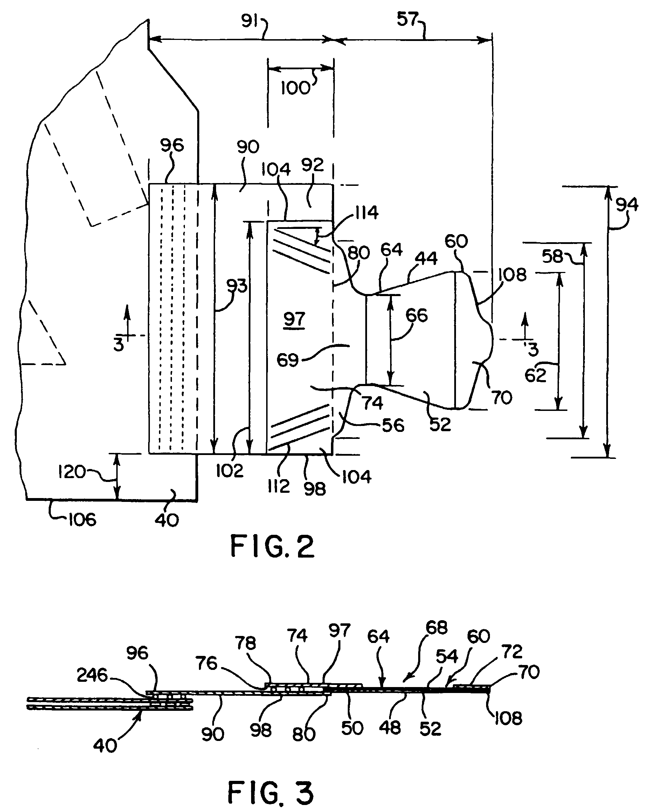

With reference to Figs. 1 and 2, a representative article of the invention, such as disposable diaper 20, is shown in its fully extended condition with all of the elasticized gathers stretched out and removed. The article has a first waistband section, such as rear waistband section 40, a second waistband section, such as front waistband section 38, and an intermediate section 42 which interconnects the first and second waistband sections. The article comprises a backsheet layer 22, and a pair of side panels 90, each of which extends laterally from opposed lateral ends of at least one waistband section of backsheet 22. Each of the side panels includes a terminal free end region 92 which has a predetermined length dimension 94 thereof. Each side panel also has a width 91 and a base length 93. A stress beam section 98 is connected to each of the side panels 90 along its free end region 92, and the stress beam section provides for a relatively high Gurley stiffness value, such as a Gurley stiffness value of at least about 20 mg. The stress beam section also has a length dimension 102 which is at least a significant substantial percentage, such as about 33 percent, of the length 94 of the free end region 92 of the side panel. A fastening tab 44 is connected to each of the stress beam sections and is arranged to extend laterally from each of the side panels 90 for securing the waistband sections of the article about a wearer during the use of the article. In particular configurations of the invention, the fastening tab can have a base length 58 which is not more than a selected limited percentage, such as about 90 percent, of the length 102 of the stress beam section 98.

With reference to Figs. 1 and 2, a representative article of the invention, such as disposable diaper 20, has a first waistband section, such as rear waistband section 40, a second waistband section, such as front waistband section 38, and an intermediate section 42 which interconnects the first and second waistband sections. The article comprises a backsheet layer 22, and a fastening means, such as fastening tab 44, operably connected to opposed lateral ends of at least one waistband portion 40 or 38 of the backsheet layer for securing the waistband sections of the article about a wearer during the use of the article. The fastening means has a factory bond section 50, a user bond section 52, and a seam section 69 which is located between the factory bond and user bond sections. The user bond section has a length dimension, such as fastening tab length 62, which is larger than a length dimension of said seam section. Depending upon the particular tab configuration, the seam section length may correspond to the base length 58 or the intermediate length 66 of the fastening tab, as appropriate.

Yet another aspect of the invention provides a fastener article which comprises a tab substrate 48 having a factory bond section 50, a user bond section 52, and a seam section 69 which is located between the factory bond and user bond sections. The user bond section has a length dimension, such as fastening tab length 62, which is larger than a length dimension of the seam section. Depending upon the particular tab configuration, the seam section length may correspond to the base length 58 or to the intermediate length 66 of the fastener tab, as appropriate.

In the various configurations of the invention, diaper 20 can further include a liquid permeable topsheet layer 24 superposed in facing relation with the backsheet layer, and an absorbent body 26 interposed between the backsheet and topsheet layers.

Diaper 20 defines a longitudinally extending length dimension 86 and a laterally extending width dimension 88, as representatively shown in Fig. 1, and may have any desired shape, such as rectangular, I-shaped, a generally hourglass shape, or a T-shape. With the T-shape, the crossbar of the "T" may comprise the front waistband portion of the diaper or may alternatively comprise the rear waistband portion of the diaper.

Backsheet 22 can generally provide an outer cover member of the article and may be composed of a liquid permeable material, but preferably comprises a material which is configured to be substantially impermeable to liquids. For example, a typical backsheet can be manufactured from a thin plastic film, or other flexible liquid-impermeable material. As used in the present specification, the term "flexible" refers to materials which are compliant and which will readily conform to the general shape and contours of the wearer's body. Backsheet 22 prevents the exudates contained in absorbent body, 26 from wetting articles, such as bedsheets and overgarments, which contact diaper 20. In particular embodiments of the invention, backsheet 22 is a polyethylene film having a thickness of from about 0.012 millimeters (0.5 mil) to about 0.051 millimeters (2.0 mils). In the shown embodiment, the backsheet is a film having a thickness of about 0.0254 mm - 0.0381 mm (about 1 - 1.5 mil). For example, the backsheet film can have a thickness of about 0.03175 mm (about 1.25 mil). Alternative constructions of the backsheet may comprise a woven or nonwoven fibrous web layer which has been totally or partially constructed or treated to impart the desired levels of liquid impermeability to selected regions that are adjacent or proximate the absorbent body. Backsheet 22 typically provides the outer cover of the article. Optionally, however, the article may comprise a separate outer cover member which is in addition to the backsheet.

Backsheet 22 may alternatively be composed of a micro-porous, "breathable" material which permits gases, such as water vapor, to escape from absorbent body 26 while substantially preventing liquid exudates from passing through the backsheet. For example, the breathable backsheet may be composed of a microporous polymer film or a nonwoven fabric which has been coated or otherwise treated to impart a desired level of liquid impermeability. For example, a suitable microporous film can be a PMP-1 material, which is available from Mitsui Toatsu Chemicals, Inc., a company having offices in Tokyo, Japan; or an XKO-8044 polyolefin film available from 3M Company of Minneapolis, Minnesota. The backsheet may also be embossed or otherwise be provided with a matte finish to exhibit a more aesthetically pleasing appearance.

The size of backsheet 22 is typically determined by the size of absorbent body 26 and the particular diaper design selected. Backsheet 22, for example, may have a generally T-shape, a generally I-shape or a modified hourglass shape, and may extend beyond the terminal edges of absorbent body 26 by a selected distance, such as a distance of at least about 1.27 cm (about 0.5 in). In particular embodiments of the invention, backsheet can extend beyond the edges of absorbent body 26 by a distance within the range of about 1.3 centimeters to 2.5 centimeters (about 0.5 to 1.0 inch). Topsheet 24 presents a body-facing surface which is compliant, soft-feeling, and non-irritating to the wearer's skin. Further, topsheet 24 can be less hydrophilic than absorbent body 26, and is sufficiently porous to be liquid permeable, permitting liquid to readily penetrate through its thickness to reach the absorbent body. A suitable topsheet 24 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. Topsheet 24 is typically employed to help isolate the wearer's skin from liquids held In absorbent body 26

Various woven and nonwoven fabrics can be used for topsheet 24. For example, the topsheet may be composed of a meltblown or spunbonded web of polyolefin fibers. The topsheet may also be a bonded-carded-web composed of natural fibers, synthetic fibers or combinations thereof.

For the purposes of the present description, the term "nonwoven web" means a web of material which is formed without the aid of a textile weaving or knitting process. The term "fabrics" is used to refer to all of the woven, knitted and nonwoven fibrous webs.

The topsheet fabrics may be composed of a substantially hydrophobic material, and the hydrophobic material may optionally be treated with a surfactant or otherwise processed to impart a desired level of wettability and hydrophilicity. In a particular embodiment of the invention, topsheet 24 is a nonwoven, spunbond polypropylene fabric composed of about 2.8 - 3.2 denier fibers formed into a web having a basis weight of about 22 gsm and density of about 0.06 gm/cc. The fabric is surface treated with about 0.28% Triton X-102 surfactant.

In the shown embodiment of diaper 20, for example, topsheet 24 and backsheet 22 can be generally coextensive and have length and width dimensions which are generally larger than the corresponding dimensions of absorbent body 26. Topsheet 24 is associated with and superimposed on backsheet 22, thereby defining the periphery of diaper 20.

Topsheet 24 and backsheet 22 are connected or otherwise associated together in an operable manner. As used herein, the term "associated" encompasses configurations in which topsheet 24 is directly joined to backsheet 22 by affixing topsheet 24 directly to backsheet 22, and configurations wherein topsheet 24 is indirectly joined to backsheet 22 by affixing topsheet 24 to intermediate members which in turn are affixed to backsheet 22. Topsheet 24 and backsheet 22 can be affixed directly to each other in the diaper periphery by attachment means (not shown) such as adhesive bonds, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet 24 to backsheet 22. It should be readily appreciated that the above-described attachment means may also be employed to interconnect and assemble together the other component parts of the article.

Absorbent body 26 can comprise an absorbent pad composed of selected hydrophilic fibers and high-absorbency particles. The absorbent body is positioned between topsheet 24 and backsheet 22 to form diaper 20. The absorbent body has a construction which is generally compressible, conformable, non-irritating to the wearer's skin, and capable of absorbing and retaining liquid body exudates. It should be understood that, for purposes of this invention, the absorbent body may comprise a single, integral piece of material, or alternatively, may comprise a plurality of individual separate pieces of material which are operably assembled together.

Various types of wettable, hydrophilic fibrous material can be-used to form the component parts of absorbent body 26. Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers; and synthetic fibers composed of a nonwettable thermoplastic polymer, such as polypropylene fibers, which have been hydrophilized by appropriate means. The fibers may be hydrophilized, for example, by treatment with silica, treatment with a material which has a suitable hydrophilic moiety and is not readily removable from the fiber, or by sheathing the nonwettable, hydrophobic fiber with a hydrophilic polymer during or after the formation of the fiber. For the purposes of the present invention, it is contemplated that selected blends of the various types of fibers mentioned above may also be employed.

As used herein, the term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System. When measured with this system in accordance with the procedure described in detail herein below, fibers having contact angles less than 90° are designated "wettable", while fibers having contact angles greater than 90° are designated "nonwettable".

Absorbent body 26 can comprise a matrix of hydrophilic fibers, such as a web of cellulosic fluff, mixed with particles of high-absorbency material. In particular arrangements, absorbent body 26 may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed for example, the concentrations of superabsorbent particles may be arranged in a non-step-wise gradient through a substantial portion of the thickness (z-direction) of the absorbent structure, with lower concentrations toward the bodyside of the absorbent body and relatively higher concentrations toward the outerside of the absorbent structure. Suitable z-gradient configurations are described in US-A-4699823 issued October 13, 1997 to Kellenberger et al.

Alternatively, the concentrations of superabsorbent particles may be arranged in a non-step-wise gradient, through a substantial portion of the thickness (z-direction) of the absorbent structure, with higher concentrations toward the bodyside of the absorbent body and relatively lower concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

The high-absorbency material may comprise absorbent gelling materials, such as superabsorbents. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces.

Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alpha-olefins, poly(vinyl pyrrolidone), poly(vinylmorpholinone), poly(vinyl alcohol), and mixtures and copolymers thereof. Further polymers suitable for use in the absorbent body include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be useful in the present invention. Other suitable absorbent celling materials are disclosed by Assarsson et al. in US-A-3901236 issued August 26, 1975. Processes for preparing synthetic absorbent gelling polymers are disclosed in US-A-4076663 issued February 28, 1978 to Masuda et al. and US-A-4286082 issued August 25, 1981 to Tsubakimoto et al.

Synthetic absorbent gelling materials typically are xerogels which form hydrogels when wetted. The term "hydrogel", however, has commonly been used to also refer to both the wetted and unwetted forms of the material.

As mentioned previously, the high-absorbency material used in absorbent body 26 is generally in the form of discrete particles. The particles can be of any desired shape, for example, spiral or semi-spiral, cubic, rod-like, polyhedral, etc. Shapes having a large greatest dimension/smallest dimension ratio, like needles, flakes, and fibers, are also contemplated for use herein. Conglomerates of particles of absorbent gelling material may also be used in absorbent body 26.

Preferred for use are particles having an average size of from about 20 microns to about 1 millimeter. "Particle size" as used herein means the weighted average of the smallest dimension of the individual particles.

The hydrophilic fibers and high-absorbency particles can be configured to form an average composite basis weight which is within the range of about 400 - 900 gsm. In certain aspects of the invention, the average composite basis weight is within the range of about 500-800 gsm, and preferably is within the range of about 550-750 gsm to provide desired performance.

To improve the containment of the high-absorbency material, absorbent body 25 can include an improved overwrap, such as wrap sheet 28, placed immediately adjacent and around absorbent body 26. The wrap sheet is preferably a layer of absorbent material which covers the major bodyside and outerside surfaces of the absorbent body, and preferably encloses substantially all of the peripheral edges of the absorbent body to form a substantially complete envelope thereabout. Alternatively, the wrap sheet can provide an absorbent wrap which covers the major bodyside and outerside surfaces of the absorbent body, and encloses substantially only the lateral side edges of the absorbent body. Accordingly, both the linear and the inwardly curved portions of the lateral side edges of the wrap sheet would be closed about the absorbent body. In such an arrangement, however, the end edges of the wrap sheet may not be completely closed around the end edges of the absorbent body at the waistband regions of the article.

For example, the complete wrap sheet 28, or at least the bodyside layer of the wrap sheet, may comprise a meltblown web composed of meltblown polypropylene fibers having a fiber size of about 5 micrometers and arranged to form a basis weight within the range or about 8-20 gsm.

Another example of absorbent wrap 28 may comprise a low porosity cellulosic tissue web composed of an approximately 50/50 blend of hardwood/softwood fibers. The tissue has a 5.89 kg (13 lb) basis weight at the reel and porosity of about 969 cts/m² (about 90 cts/sq. ft.).

Absorbent wrap 28 may comprise a multi-element wrapsheet which includes a separate bodyside wrap layer 30 and a separate outerside wrap layer 32, each of which extends past all or some of the peripheral edges of absorbent body 26. Such a configuration of the wrap sheet can, for example, facilitate the formation of a substantially complete sealing and closure around the peripheral edges of absorbent body 26. In the back waistband portion of the illustrated diaper, the absorbent wrap may also be configured to extend an increased distance away from the periphery of the absorbent body to add opacity and strength to the back side-sections of the diaper. In the illustrated embodiment, the bodyside and outerside layers of absorbent wrap 28 extend at least about 1.27 cm (about 1/2 inch) beyond the peripheral edges of the absorbent body to provide an outwardly protruding, flange-type bonding area over which the periphery of the bodyside portion of the absorbent wrap may be completely or partially connected to the periphery of the outerside portion of the absorbent wrap.

The bodyside and outerside layers of wrap sheet 28 may be composed of substantially the same material, or may be composed of different materials. For example, the outerside layer of the wrap sheet may be composed of a relatively lower basis weight material having a relatively high porosity, such as a wet strength cellulosic tissue composed of softwood pulp. The bodyside layer of the wrap sheet may comprise one of the previously described wrap sheet materials which has a relatively low porosity. The low porosity bodyside layer can better prevent the migration of superabsorbent particles onto the wearer's skin, and the high porosity, lower basis weight outerside layer can help reduce costs.

Diaper 20 can also include a surge management layer 84 which helps to decelerate and diffuse surges of liquid that may be introduced into the absorbent body of the article. In the illustrated embodiment, for example, surge layer 84 can be located on an inwardly facing body side surface of topsheet layer 24. Alternatively, surge layer 84 may be located adjacent to an outer side surface of topsheet 24. Accordingly, the surge layer would then be interposed between topsheet 24 and absorbent body 26.

Leg elastic members 34 are located in the lateral side margins 110 of diaper 20 and are arranged to draw and hold diaper 20 against the legs of the wearer. The elastic members are secured to diaper 20 in an elastically contractible condition so that in a normal under strain configuration, the elastic members effectively contract against diaper 20. The elastic members can be secured in an elastically contractible condition in at least two ways, for example, the elastic members may be stretched and secured while diaper 20 is in an uncontracted condition. Alternatively, diaper 20 may be contracted, for example, by pleating, and the elastic members secured and connected to diaper 20 while the elastic members are in their unrelaxed or unstretched condition. Still other means, such as heat-shrink elastic material, may be used to gather the garment.

In the embodiment illustrated in Fig. 1, leg elastic members 34 extend essentially along the complete length of the intermediate crotch region 42 of diaper 20. Alternatively, elastic members 34 may extend the entire length of diaper 20, or any other length suitable providing the arrangement of elastically contractible lines desired for the particular diaper design.

Elastic members 34 may have any of a multitude of configurations. For example, the width of the individual elastic members 34 may be varied from 0.25 millimeters (0.01 inches) to 25 millimeters (1.0 inches) or more. The elastic members may comprise a single strand of elastic material, or may comprise several parallel or non-parallel strands of elastic material, or may be applied in a rectilinear or curvilinear arrangement. Where the strands are non-parallel, two or more of the strands may intersect or otherwise interconnect within the elastic member. The elastic members may be affixed to the diaper in any of several ways which are known in the art. For example, the elastic members may be ultrasonically bonded, heat and pressure sealed using a variety of bonding patterns, or adhesively bonded to diaper 20 with sprayed or swirled patterns of hotmelt adhesive.

In the illustrated embodiments of the invention, leg elastic members 34 may comprise a carrier sheet (not shown) to which are attached a grouped set of elastics composed of a plurality of individual elastic strands 39. The elastic strands may intersect or be interconnected, or be entirely separated from each other. The shown carrier sheet may, for example, comprise a 0.002 cm thick film of unembossed polypropylene material. The shown elastic strands can, for example, be composed of "LYCRA" elastomer available from DuPont, a business having offices in Wilmington, Delaware. Each elastic strand is typically within the range of about 470 - 1500 decitex (dtx), and may be about 940 - 1050 dtx. In particular embodiments of the invention, for example, three or four strands can be employed for each elasticized legband.

In addition, leg elastics 34 may be generally straight or optionally curved. For example, the curved elastics can be inwardly bowed toward the longitudinal centerline of the diaper with the innermost point (or apex, relative to the cross-direction of the article) of the set of curved elastic strands positioned approximately 0.75 - 1.5 inches inward from the outer most edge of the set of elastic strands. In particular arrangements, the curvature of the elastics may not be configured or positioned symmetrically relative to the lateral centerline of the diaper. The curved elastics may have an inwardly bowed and outwardly bowed, reflex-type of curvature, and the length-wise center of the elastics may optionally be offset by a selected distance within the range of about 0 - 8 cm toward either the front or rear waistband of the diaper to provide desired fit and appearance. In particular embodiments of the invention, the innermost point (apex) of the set of curved elastics can be offset about 0 - 12 cm towards the front or rear waistband of the diaper, and the outwardly bowed reflexed-portion can be positioned toward the diaper front waistband.

In the shown embodiment, diaper 20 includes a waist elastic 36 positioned in the longitudinal margins of either or both of front waistband 38 and rear waistband 40. The waist elastics may be composed of any suitable elastomeric material, such as an elastomer film, an elastic foam, multiple elastic strands, an elastomeric fabric or the like. For example, suitable elastic waist constructions are described in US-A-4916005 to Lippert Diaper 20 can also include a pair of elasticized containment flaps 82 which extend longitudinally along the length dimension 86 of the diaper. The containment flaps are typically positioned laterally inboard from leg elastics 34, and substantially symmetrically placed on each side of the lengthwise, longitudinal centerline of the diaper. Examples of suitable containment flap constructions are described in US-A-4704116 issued November 3, 1987, to K. Enloe. The containment flaps may be composed of a wettable or a non-wettable material, as desired. In addition, the containment flap material may be permeable to gas or permeable to both gas and liquid.

In an optional, alternative embodiment of the invention, diaper 20 may include elasticized waist flaps, such as those described in US-A-4753646 issued June 28, 1988, to K. Enloe. Similar to the construction of the containment flaps, the waist flaps may be composed of a wettable or non-wettable material, as desired. The waist flap material may be permeable to gas, or permeable to both gas and liquid.

Absorbent article structures suitable for use with the present invention are described in US-A-5192606 issued March 9, 1993. Other absorbent article structures suitable for use with the present invention are described in US-A-5509915.

To provide a refastenable adhesive taping system, diaper 20 can include a supplemental landing zone patch 46, which provides a target zone for receiving an adhesive attachment of tape fasteners 44 thereon. In the illustrated embodiment of the invention, landing zone patch 46 is positioned on the outward surface of backsheet 22 and is located on the second, front waistband portion 38 of the diaper. Landing zone patch 46 is constructed of a suitable material, such as polypropylene, polyester, or the like, and is configured and arranged to accept a secure adhesion of tape fasteners 44. In addition, the landing zone patch and the tape fasteners are cooperatively constructed and arranged to provide a releasable adhesion which allows the tape fastener to be removed from the landing zone patch for repositioning and re-adhesion without tearing or excessively deforming the material of backsheet 22. For example, a suitable tape landing zone construction is described in US-A-5,024,672 issued June 18, 1991, to L. Widlund. A further construction of a tape landing zone patch is described in US-A-4,753,649 issued to Pazdernik.

In various embodiments of the invention, a tape fastener 44 can be located at either or both of lateral end regions 116 and 118 of either or both of waistbands 38 and 40, respectively. The representatively shown embodiment has the tape fasteners located at the terminal side edges of rear waistband 40.

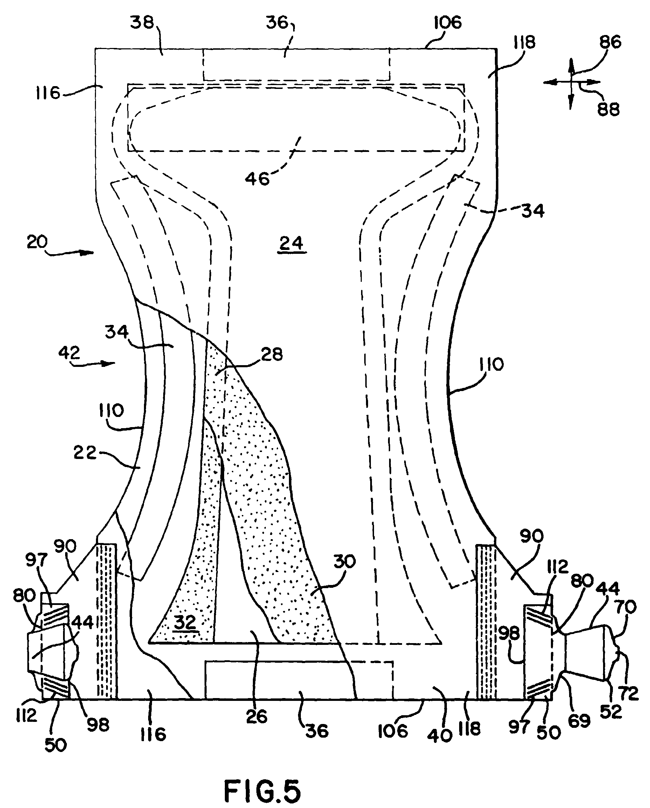

With reference to Figs. 1 and 5, each side panel 90 extends laterally from the opposed lateral ends of at least one waistband portion of backsheet 22, such as rear waistband portion 40, to provide terminal side sections of the article. In addition, each side panel can substantially span from a laterally extending, terminal waistband edge 106 to approximately the location of a corresponding leg opening section of the diaper. Diaper 20, for example, has a laterally opposed pair of leg openings formed by appointed, medial sections of the shown pair of longitudinally extending, side edge regions 110.

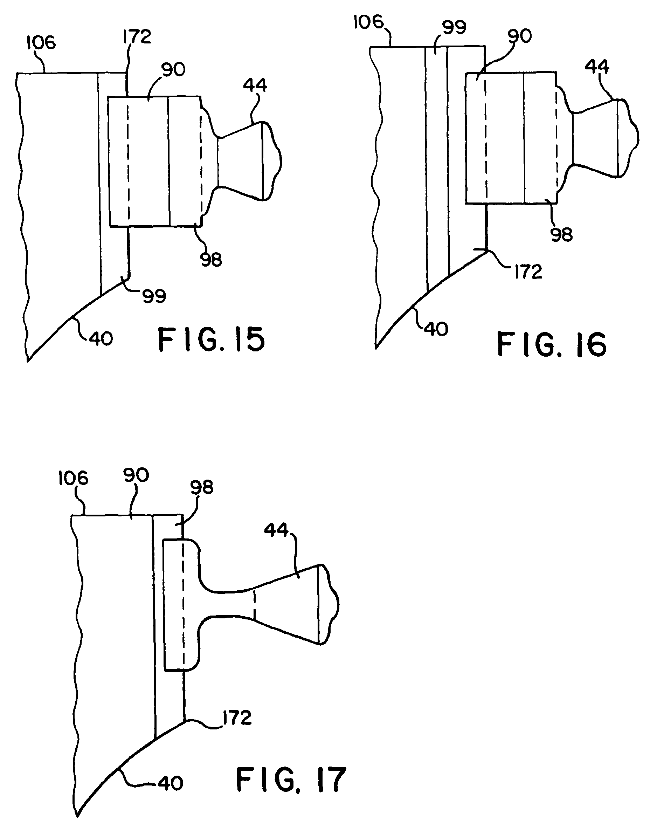

In the various configurations of the invention, the side panels may be integrally formed with a selected diaper component. For example, side panels 90 can be integrally formed from the layer of material which provides backsheet layer 22, or may be integrally formed from the material employed to provide topsheet 24 (e.g. Fig. 17). In alternative configurations, the side panels 90 may be separate members that are connected to backsheet 22, to topsheet 24, in between the backsheet and topsheet, or combinations thereof.

In particular aspects of the invention, each of the side panels 90 may be formed from a separate piece of material which is then suitably assembled and attached to the selected front and/or rear waistband portion of the diaper article. In the illustrated embodiments of the invention, for example, side panels 90 are attached to the rear waistband portion of backsheet 22, and can be operably attached to either or both of the backsheet and topsheet components of the article. The side panels extend laterally to form a pair of opposed waist-flap sections of the diaper, and are attached with suitable connecting means, such as adhesive bonding, thermal bonding, ultrasonic bonding, clips, staples, sewing or the like.

Side panels 90 may be composed of a substantially non-elastomeric material, such as polymer films, woven fabrics, nonwoven fabrics or the like, as well as combinations thereof. In particular aspects of the invention, side panels 90 are composed of a substantially elastomeric material, such as a stretch-bonded-laminate (SBL) material, a neck-bonded-laminate (NBL) material, an elastomeric film, in elastomeric foam material, or the like. For example, suitable meltblown elastomeric fibrous webs for forming side panels 90 are described in US-A-4,663,220 issued May 5, 1987 to T. Wisneski et al.

Examples of composite fabrics comprising at least one layer of nonwoven textile fabric secured to a fibrous elastic layer are described in European Patent Application EP-A-0 110 010 published on April 8, 1987 with the inventors listed as J. Taylor et al.. Examples of NBL materials are described in US-A-5226992 issued July 13, 1993 to Mormon.

As previously mentioned, various suitable constructions can be employed to attach the side panels 90 to the selected waistband portions of the article. Where the side panels are composed of an elastomeric material, for example, suitable constructions for securing a pair of elastomeric, stretchable members to the lateral, side portions of an article to extend laterally outward beyond the opposite side regions of the outer cover and liner components of an article can be found in US-A-4938753 issued July 3, 1990 to P. VanGompel et al.

In other aspects of the invention, side panels 90 can be composed of a material having a Gurley stiffness value of not more than about 10,000 milligrams (mg). Optionally, the side panel material has a stiffness value of not more than about 2,000 mg, and optionally has a stiffness value of not more than about 200 mg.

In further aspects of the invention, side panels 90 can be composed of a material having a Gurley stiffness value of not less than about 1 mg. Alternatively, the side panel material has a stiffness value of not less than about 4 mg, and optionally has a stiffness value of not less than about 8 mg.

In the various configurations of the invention the desired Gurley stiffness value can be exhibited with respect to the width dimension, or with respect to both the width and length dimensions of the side panel.

In particular configurations of the invention where side panels 90 are composed of an elastomeric material, the elastomeric side panels are composed of a material which can provide an elongation at peak load of at least about 30 percent when subjected to a tensile force load of about 0.58 N/cm of the sample dimension that is measured perpendicular to the direction of the applied load (about 0.33 pounds per lineal inch). Alternatively, the elastomeric side panel material can provide an elongation of at least about 100 %, and optionally can provide an elongation of at least about 300 % to provide desired performance.

In conventional fastening systems, the fastening stress is applied to the factory bond between fastening tab 44 and the side sections or rear waistband 40 substantially across the base length 58 of the fastening tab. As a result, relatively low levels of stress are applied to the regions of the ear sections that are longitudinally adjacent to the side edges of the fastening tab. As a result, the longitudinally adjacent regions tend to wrinkle and curl away from the body of the wearer. The wrinkling and curling can be unsightly and can create gaps along the waistband and along the leg opening region of the diaper through which waste materials may leak from the diaper. Attempts to address this problem have employed complex fastening systems which extend along substantially the entire free edge length of the ear sections of the article. Other attempts to address this problem have employed multiple fastening tapes or a large, wide fastening tab. The wide fastening tabs or tapered fastening tabs have transmitted excessive stresses to the user-bond securement section of the fastening system. Such stresses can tend to undesirably disconnect the user bond portion of the fastening system when the wearer shifts and moves about. In addition, such configurations may not sufficiently conform and adjust to the movements of the wearer, and can result in excessive irritation of the wearer's skin.

To help address the problems associated with conventional fastening systems such as those described above, the present invention can advantageously include a distinctive reinforcement, stress beam section 98. The stress beam can disperse and dissipate the fastening forces across the length of each side panel 90. In addition, the stress beam section can provide for a sufficient stiffening and reinforcement of its associated waistband section to help prevent undesired and excessive wrinkling, necking-down or folding-over of the lateral end of the waistband or side panel during the use of the article.

In the various configurations of the invention, stress beam section 98 can be integrally formed from the same material employed to form the side panel 90 associated therewith. For example, a portion of the free end of a side panel may be doubled over one or more times along longitudinally extending fold lines to generate an operable stress beam section. Alternatively, the stress beam section can be provided by densifying or embossing a selectively sized and shaped region of side panel 90 to an extent which provides operable levels of strength and stiffness.

In other arrangements of the invention, stress beam section 98 can include a stiffening or reinforcement member provided by a selectively shaped and sized region of material which is integrally formed with fastening tab substrate 48. Alternatively, the stress beam section can include a separate stiffening or reinforcement member 97 which is appropriately configured, and is assembled to the free end region of the side panel. For example, the stress beam section can be provided for by a suitably sized and shaped piece of material attached to a suitable surface of each side panel 90, such as an inward bodyside surface of each panel. The material may be composed of a polymer film, a nonwoven fabric, a woven fabric or the like, as well as combinations thereof. In a particular configuration, the stress beam section can include a stiffening member composed of the material employed to construct release tape material 74 and/or fastening tab substrate 48. In the various configurations of the invention the stress beam section can be substantially non-extensible and/or substantially non-elastomeric.

With reference to Fig. 2, a stress beam section 98 can be operably connected to each side panel 90 along the free end region 92 of the side panel with suitable attaching means, such as adhesive bonding, thermal bonding, ultrasonic bonding, clips, staples, sewing or the like. The stress beam section has a laterally extending, cross-directional width dimension 100 and a longitudinally extending length dimension 102. To obtain desired performance, it can be advantageous to position stress beam section 98 at a medial location along the length of side panel 90. In the shown embodiment, for example, the stress beam section is substantially centered along the longitudinal length of the free end section of the side panel.

In a particular aspect of the invention, the stress beam section length 102 is at least about 33 percent of the length 94 of the free end region 92 of side panel 90. Alternatively, the stress beam section length is at least about 80 percent of the free end region length 94 of the side panel, and optionally is about 100 percent of the free end region length to provide desired benefits. Particular configurations of the invention can include a stress beam having a length of up to about 125% of the free end region length 94 of the side panel to provide desired performance. In other aspects of the invention, the stress beam section length is not less than about 1.25 cm. Alternatively, the stress beam section length is not less than about 2.5 cm, and optionally is not less than about 5 cm to provide improved performance. In further aspects of the invention, the stress beam section length is not more than about 15 cm. Alternatively, the stress beam section length is not more than about 13 cm, and optionally is not more than about 10 cm to provide desired performance.

In the various configurations of the invention, the stress beam section width 100 is not less than about 0.1 cm. Alternatively, the stress beam section width is not less than about 0.5 cm, and optionally is not less than about 1.0 cm to provide improved performance. In other aspects of the invention, the stress beam section width is not more than about 10 cm. Alternatively, the stress beam section width is not more than about 5 cm, and optionally is not more than about 2.5 cm to provide desired performance.

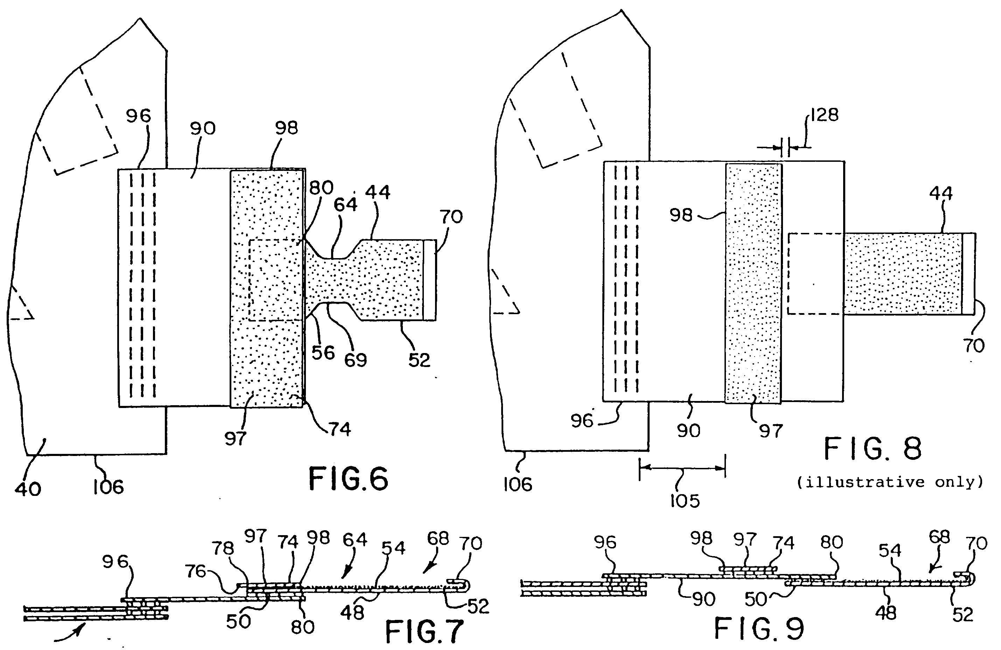

A particular aspect of the invention can be configured to employ a separate piece of material which operatively forms a member that overlaps the material of side panel 90 to provide for the desired stress beam section 98. In the representative configurations shown in Fig. 2 and 6, for example, substantially 100% of the width of the separate beam member can be arranged to overlap the material of side panel 90.

While a preferred construction of the invention can have stress beam section 98 connected directly to fastener tab 44, the various aspects of the invention can include configurations wherein the stress beam section is a discrete component that is spaced from and indirectly connected to the terminal end of the fastener tab by an intervening section of material.
The stress beam section 98 can also be located on side panel 90 at a position that is intermediate fastener tab 44 and absorbent body (26). This is shown in Fig. 8 which does not show, however, the inventive concept according to the present invention. In particular aspects, the spacing distance 105 between the edge of the attached section 96 of side panel 90 and the relatively closest edge of its associated stress beam section is within the range of about 0.1 - 12.4 cm, and is optionally about 2.5 cm to provide desired performance. In other aspects of the invention, the gap distance 128 between the factory bond end of fastener tab 44 and the relatively closest edge of its associated stress beam section is within the range of about 0.1 - 2.54 cm.

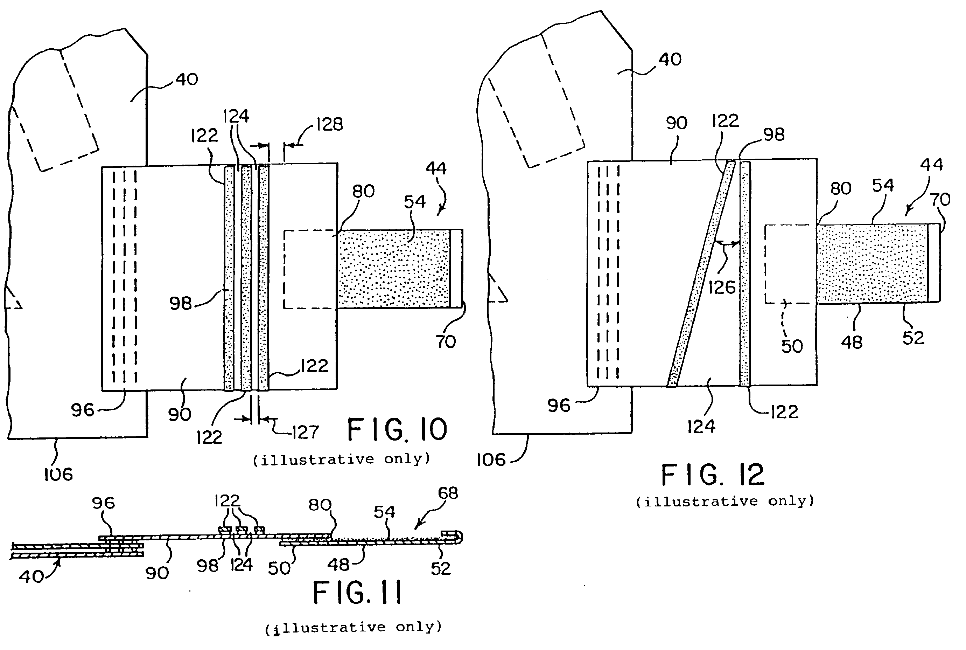

The various configurations of the invention can comprise a stress beam section 98 which includes a plurality of individual beam elements 122. Fig. 10 representatively shows such a plurality of individual beam elements without illustrating the inventive concept. When the beam elements are generally parallel and approximately of the same length, for example, the multiple elements can effectively function as a single beam section 98. A multi-element beam section can be distinctively designed and configured to provide a selective control and distribution of stresses through its associated side panel member 90. For example, the multi-element beam section can include individual elements spaced apart by a selected separation distance 127. The separation distances can advantageously provide flexure regions 124 between the individual beam elements. In the shown aspect, the flexure regions can provide longitudinally extending flexure areas which allow increased flexibility and pivotability about those areas.

As representatively shown in Fig. 10 which does not show the inventive concept, operable flexure joints are provided between the individual, parallel beam elements, thereby creating controlled areas along which the composite beam section can operably fold and flex in a resilient manner. In the shown aspect for example, the separation distance 127 between the spaced apart beam elements can be within the range of about 1 - 10 mm.

Another aspect of the invention, representatively shown in Fig. 12 which does not show the inventive concept, can include a side panel 90 having a multi-element beam section wherein the individual beam elements 122 are positioned at a selected angle with respect to each another. The various configurations of the invention can have the angle 126 between beam elements arranged to be within the range of about 1 - 179 degrees. In particular aspects the angle 126 is within the range of about 1 - 89 degrees, and optionally is within the range of about 1 - 44 degrees to provide desired performance.

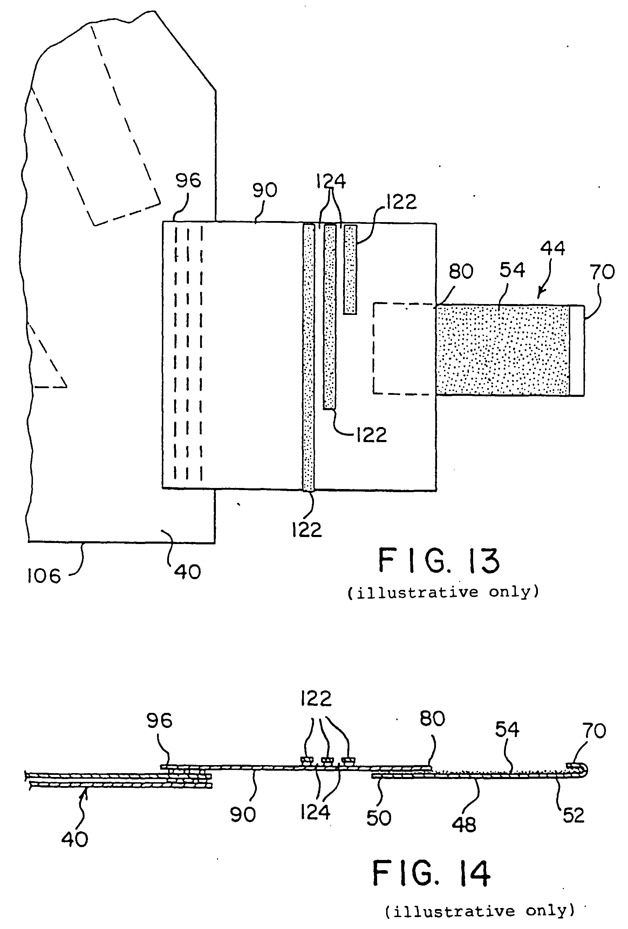

Further aspects of the invention can incorporate a multi-element beam section wherein the individual beam elements 122 have different lengths, as representatively shown in Fig. 13 which does not show the inventive concept. The length of each individual beam element 122 can be a selected proportion of the length of the free edge of its associated side panel member 90. The length of a beam element can be as much as 100 % of the free edge length or as little as 5 % of the free edge length. In the illustrated aspect, for example, the beam elements are arranged with graduated lengths of increasing size, as one moves away from fastening tab 44. Alternatively, the beam elements can be arranged with graduated lengths of decreasing size, as one moves away from fastening tab 44, and optionally can be arranged in other patterns depending upon the desired pattern of flexure regions 124.

In particular aspects of the invention, stress beam section 98 extends along the longitudinal length of side panel 90 to be substantially coterminous with the laterally extending waistband edge 106 of the article. In the illustrated embodiment, fastening tab 44 is approximately centered along the length of stress beam section 98. Alternatively, the location of fastening tab 44 may be offset longitudinally of the diaper by a selected distance away from the lengthwise center of stress beam section 98. In particular aspects of the invention, fastening tab 44 may be spaced from waistband edge 106 by a spacing distance 120 which is not more than about 6 centimeters. Alternatively the spacing is not more than about 4 centimeters, and optionally is not more than about 2 centimeters to provide improved benefits. In further aspects of the invention, the edge of fastening tab 44 may be arranged to substantially coincide with waistband edge 106 to provide improved performance.

In the various aspects of the invention, stress beam section 98 can provide for a rigidity, stiffness value which is greater than the stiffness value of side panel 90. More particularly, the stress beam section can advantageously be composed of a material which provides for a Gurley stiffness value of the stress beam of at least about 20 mg, and in desired configurations, can provide for a Gurley stiffness value of at least about 100 mg. Alternatively, the material of stress beam section 98 provides for a stiffness value of at least about 200 mg, and optionally, provides for a stiffness value of at least about 400 mg.

If the stress beam section is too stiff, however, it can cause excessive irritation and red-marking of the wearer's skin. Accordingly, further aspects of the invention can be configured with the material of stress beam section 98 providing for a Gurley stiffness value of the stress beam not more than about 50,000 mg. Alternatively, the stress beam material can provide for a stress beam stiffness value of not more than about 10,000 mg, and optionally can provide for a stiffness value of not more than about 1,000 mg to provide desired performance.

In the various configurations of the invention the desired Gurley stiffness value can be exhibited with respect to the length dimension, or with respect to both the width and length dimensions of the stress beam section.

In further aspects of the invention, the assembled stress beam section 98, relative to its associated side panel 90 connected thereto, exhibits a stiffness ratio of at least about 5:1. Alternatively, this stiffness ratio is at least about 10:1, and optionally is at least about 30:1. In other aspects of the invention, stress beam section 98 and its associated side panel 90 have a stiffness ratio of not more than about 50,000:1. Alternatively, the stiffness ratio is not more than about 5,000:1, and optionally is not more than about 500:1 to provide desired benefits.

In the various configurations of the invention, stress beam 98 can include at least one resilient, controlled flexure, hinge region 112. In the shown embodiment, the stress beam includes a plurality of generally laterally extending resilient hinges 112. In particular aspects of the invention, hinge regions 112 can optionally be angled a selected number of degrees from a line that is substantially parallel to cross-directional width dimension 88. The resultant offset angle 114 can be within the range of about -40° to about +40°. Alternatively, the offset angle can be within the range of about 0° - 40°, and optionally, can be within the range of about 10° - 30° to provide improved benefits.

Hinge regions 112 can be configured with a relatively lower rigidity and stiffness, as compared to the other regions of stress beam 98. After flexing in response to an applied load imparted by the wearer, the selected resilience provided by a structured spring action exhibited by hinge regions 112 can provide an operable recovery force which substantially unflexes the hinge region upon the removal of the applied load.

A fastening means, such as fastening tape tab 44 is operably connected to each of the side panels 90. In the illustrated configuration, the juncture section along which fastening tab 44 intersects the terminal side edge of panel 90 provides a relatively narrowed panel juncture region 80. The connection may be accomplished with suitable attaching means, such as adhesive bonding, thermal bonding, ultrasonic bonding, clips, staples, sewing or the like. Alternatively, the fastening tab substrate may be integrally formed from the material employed to form stress beam section 98. In optional configurations, the fastening tab may be directly or indirectly connected to the stress beam section 98 associated with the respective side panel. For example, the fastening tab 44 may indirectly connect to its associated stress beam 98 by way of an intervening section of side panel 90, as representatively shown in Fig. 8, which does not illustrate the invention.

In the illustrated embodiments of the invention, the components of the fastening means cooperate to secure the front and rear waistband portions of the article about a wearer. In particular, the rear waistband section of the shown embodiment overlaps the front waistband section of the article and the fastening means operably attaches to appointed regions of the front waistband portion. Fastening tab 44 has a longitudinally extending length dimension and a laterally extending width dimension. In addition, the fastening tab has a base section 56, a user bond end section 60 and an intermediate section 64 which interconnects the base and end sections. Base section 56 has a longitudinal length dimension 58, end section 60 has a longitudinal length dimension 62, and intermediate section 64 has a longitudinal length dimension 66.

In particular aspects of the invention, fastening tab 44 has, along its respective panel juncture region 80, a base length 58 which is not more than about 90 percent of the length 102 of stress beam section 98. Alternatively, the fastening tab base length is not more than about 80 percent of the stress beam section length, and optionally is not more than about 50 percent of the stress beam section length to provide desired performance. In other aspects of the invention, fastening tab 44 has a base length 58 which is not less than about 1 percent of the length 102 of stress beam section 98. Alternatively, the base length is not less than about 5 percent of the stress beam section length, and optionally is not less than about 20 percent of the stress beam section length to provide desired benefits. Accordingly, when the fastening means is employed to secure the article on the wearer, the end sections 104 of the stress beam section are not further attached to the front waistband of the article by the operation of securing the article on the wearer. As a result, the unattached end sections 104 can advantageously slide, bend and otherwise move relative to the secured portions of the article without excessively disturbing the securing attachment between the user bond section of the fastening tab and the appointed securement zone of the article.

In the illustrated embodiments length 58 of the base section 56 of fastening tab 44 is relatively larger than the length 66 of the fastening tab intermediate section 64. Alternatively, however, base length 58 may be equal to or less than the intermediate section length 66. In either case, the construction of the fastening system of the invention provides a seam section 69 of the fastening tab which is positioned between stress beam section 98 and the user bond section 52 of the fastening tab. As determined when the fastening tab is in its relaxed and substantially untensioned condition, the tab seam section generally represents the narrowest region of the fastening tab with respect to those portions of the fastening tab that are spaced from the terminal end sections of the tab. Seam section 69 can advantageously provide a relatively more flexible pivot region which can facilitate a freer, less restricted relative movement between the stress beam portion of the fastening system and user bond portion of the fastening tab. As a result, the stress beam 98 can operate to help maintain the desired waistband appearance and good fit during the movements of the wearer, and the user bond section 52 can maintain a more reliable securement with less occurrence of undesired pop-opens. The seam section can help isolate the user bond section of the fastening system from the self-adjusting movements of the side panels 90 and the stress beam sections of the fastening system. In the shown embodiment, the seam section 69 is composed of a substantially non-extensible and substantially non-elastomeric material, but may alternatively be composed of an elastomeric material which is operably assembled or otherwise incorporated into the fastening tab structure.

In particular aspects of the invention, the length of tab seam section 69 is not less than about 0.5 cm, as determined when the fastening tab is relaxed and untensioned. Alternatively, the seam section length is not less than about 1 cm, and optionally is not less than about 1.5 cm to provide desired performance. In other aspects of the invention, length of tab seam section 69 is not more than about 12.5 cm. Alternatively, the seam section length is not more than about 7 cm, and optionally is not more than about 3 cm to provide desired benefits. In the illustrated embodiments, for example, the seam length can be about 2.5 cm.

In further aspects of the invention, the ratio of the stress beam length 102 to the length dimension of the tab seam section 69 (seam ratio) is greater than 1.5:1, and alternatively is not less than about 2:1 to provide improved performance. Still other aspects of the invention incorporate a seam ratio which is not more than about 10:1, and alternatively is not more than about 7:1 to provide desire attributes. In the illustrated embodiments, for example, the seam ratio can be about 2.5:1.

In the various embodiments of the invention, fastening tab 44 can be configured to provide an adhesive fastening mechanism. More particularly, the user bond section 52 of fastening tab 44 can include a layer of primary adhesive 54 disposed across an appointed attaching surface 68 of fastening tab substrate 48. The adhesive is configured to provide a desired level of adhesion and securement when applied against the appointed landing zone region of the article. In addition, the adhesive can be configured to be capable of being removed and refastened one or more times onto the appointed landing zone region. An example of a suitable refastenable taping system is described in US-A-5147347 issued September 15, 1992 to Y. Huang et al.

In various alternative configurations of the invention, the fastening means may be provided by interlocking, mechanical-type fasteners such as hooks, buckles, snaps, buttons and the like. In particular aspects of the invention the fastening means can be provided by a hook-and-loop fastener system, a mushroom-and-loop fastener system or the like (hereinafter hook-and-loop fastener). Such fastening systems generally comprise a "hook" component and a cooperating "loop" component which engages and interlocks with the hook component. Such systems are, for example, available under the VELCRO trademark. Examples of suitable hook-and-loop fastening systems are described in US-A-5019073 issued May 28, 1991 to T. Roessler et al. In a typical configuration of a hook-and-loop fastening system, a portion of hook material is operably connected to the attaching surface 68 of fastening tab substrate 48, and the loop material is employed to construct a cooperating landing zone 46. The landing zone patch, for example, can be suitably attached to the appointed landing zone region on the outside surface of backsheet 22. An alternative configuration of a suitable hook-and-loop fastening system may have the loop material secured to the attaching surface 68 of fastening tab substrate 48. Accordingly, a region of hook material would be employed to form landing zone patch 46.

Fastening tab 44 can advantageously have a stiffness value which is different than (e.g., not more than) the stiffness value of stress beam 98. As a result, fastening tab 44 can be selectively configured with a user bond section 52 which is capable of being fastened, removed and refastened without excessively distorting or tearing the appointed landing zone region of the article. The selective tailoring of the characteristics of fastening tab 44 can be accomplished while retaining the desired stress beam characteristics of stress beam section 98. The stress beam section retains its ability to spread forces across the free end length 94 of side panel 90 without adversely affecting the fastening and refastening capability of fastening tab 44.

In particular aspects of the invention, fastening tab 44 is composed of a material which provides for a Gurley stiffness value of not more than about 500 mg. Alternatively, the fastening tabs have a stiffness value of not more than about 150 mg, and optionally have a stiffness value of not more than about 100 mg. In further aspects of the invention, fastening tab 44 has a Gurley stiffness value of not less than about 5 mg. Alternatively, the fastening tab has a stiffness value of not less than about 10 mg, and optionally has a stiffness value of not less than about 25 mg. In the various configurations of the invention the desired Gurley stiffness value can be exhibited with respect to the width dimension, or with respect to both the width and length dimensions of the fastening tab.

For the purposes of the present invention, the various rigidity stiffness values are determined with respect to a bending moment produced by a force which is directed perpendicular to the plane substantially defined by the length and width of the component being tested. A suitable technique for determining the rigidity, stiffness values described herein is a Gurley Stiffness test, a description of which is set forth in TAPPI Standard Test T 543 pm-84 (Stiffness of paper (Gurley type stiffness tester)). A suitable testing apparatus is a Gurley Digital Stiffness Tester: Model 4171-0 manufactured by Teledyne Gurley (514 Fulton Street, Troy, NY 12181-0088). This instrument allows the testing of a wide variety of materials through the use of various lengths and widths in combination with the use of a 5, 25, 50, or 200 gram weight placed in one of three positions on the pointer of the apparatus. For purposes of the present description, the stated Gurley stiffness values are intended to correspond to the values that would be generated by a "standard^{"} sized sample. Accordingly, the scale readings from the Gurley stiffness tester are appropriately converted to the stiffness of a standard size sample has a width of 2.54 cm (1") and a nominal length of 7.62 cm (3") (actual length of 8.89 cm (3.5")) The actual length of the sample is the nominal length, plus an additional 0.635 cm (0.25") of length for holding in the clamp and another 0.635 cm (0.25") of length for overlapping the vane. Tables of factors for taking scale readings generated with non-standard sized test samples and converting the readings to the stiffness of the standard size sample are given in the Instruction Manual for the Gurley Stiffness Tester provided by Teledyne Gurley. Accordingly, other designated dimensions for the test sample may also be conveniently employed, so long as the appropriate conversion factor is employed to determine the appropriate value which corresponds to the standard size sample.

In particular aspects of the invention, the user bond end section 60 of fastening tab 44 can have an end length 62 which is greater than the length 66 of the intermediate section 64 of the fastening tab, as representatively shown in Fig. 2. In the illustrated embodiment, for example, the end length can correspond to the widest length dimension of the user bond section 52 of the fastening tab. In other aspects of the invention, the length 62 of end section 60 can also be greater than the length 58 of base section 56 of the fastening tab.

More particularly, end length 62 can be at least about 10 percent greater than intermediate length 66. Alternatively, the end length can be at least about 20 percent greater than the intermediate length, and optionally can be at least about 40 percent greater than the intermediate length. In other aspects of the invention, end length 62 can be not more than about 500 percent greater than intermediate length 66. Alternatively, the end length 62 is not more than about 100 percent greater than intermediate length 66, and optionally is not more than about 60 percent greater than the intermediate length.

End length 62 can be at least about 2 percent greater than base length 58. Alternatively, end length 62 can be at least about 20 percent greater than base length 58, and optionally can be at least about 40 percent greater than the base length. In other aspects, end length 62 can be not more than about 500 percent greater than base length 58. Alternatively, end length 62 can be not more than about 100 percent greater than base length 58, and optionally is not more than about 60 percent greater than the base length of the fastening tab to provide desired performance.

In the illustrated embodiment, for example, intermediate section 64 of fastener tab 44 can be configured to provide an expanding area of the fastener tab. The expanding area provides a gradual transition between base length 58 and end length 62. To avoid the generation of excessive stress concentrations that might initiate undesired fractures, the transition area is substantially free of sharp notches or abrupt angles.

The relatively smaller base and/or intermediate lengths of tab 44 can advantageously contribute to the improved performance provided by the invention. The relatively larger length at the end portion of the user bond section 52 helps provide for a larger user bonding area which can improve the security of the fastening system. At the same time, the relatively smaller length at the base and/or intermediate portions of tab 44 can provide for a relatively greater ease of bending and/or twisting or other movement, as compared to the user bond portion of the tab. As a result, the fastening securement can be maintained at high levels while allowing substantially continual, dynamic fit adjustments at the points of interconnection between the front and rear waistband sections of the article.

With reference to Figs. 2 and 3, a tape fastener tab 44 can comprise a tape substrate member 48 having the desired fastening means, such as primary adhesive layer 54, located and disposed on a major facing surface thereof, such as surface 68. The substrate member can, for example, be composed of a fabric material or a suitable polymer film material, such as polypropylene, polyethylene or other suitable polyolefin. The material comprising substrate member 48 may be opaque, translucent or transparent, as desired, and may include graphics thereon. Optionally, the material may be tinted and/or textured, and may also be selectively embossed. In particular aspects of the invention, substrate member 48 can be constructed of a substantially non-extensible and/or substantially non-elastomeric material to provide desired benefits.

The fastener tab provides a factory-bond section 50 for connecting the tape substrate member to a selected portion of diaper 20, and a user-bond section 52 for connecting and securing the waistband sections of the diaper about the body of a wearer. In a particular aspect of the invention, the factory-bond section of fastener tab 44 is attached to the free end region 92 of side panel 90, and is constructed and configured to provide stress beam section 98. User-bond section 52 can be operably connected to a finger tab 70 which includes a substantially non-securing grasping section 72 thereof. The grasping section, in a particular aspect of the invention, can comprise a layer of absorbent material, such as a nonwoven fabric.

The factory-bond region 50 of tape fastener 44 is appointed for securement onto the desired section of its associated article during the manufacture of the article. The user-bond region 52 of tape fastener 44 is appointed for securing the article on a wearer during use. The representatively shown embodiment of the tape fastener, for example, has primary adhesive layer 54 applied onto a selected surface thereof to provide an adhesive fastening system. In the illustrated embodiment of diaper 20, the factory-bond region 50 of tape fastener 44 is attached to the lateral ends of rear waistband 40, and the user-bond region 52 of the tape fastener is employed to attach the lateral ends of rear waistband 40 to the corresponding lateral ends of front waistband 38 to secure the diaper about the waist of a child. User-bond section 52 connects to a finger tab 70 which includes a substantially non-attaching grasping section 72 thereof. The grasping section can, for example, comprise a layer of exposed absorbent material, and at least a portion of the exposed absorbent material can be operably positioned and arranged to face in the same direction as an appointed inward face of the tape fastener.

With an adhesive fastening tab, a primary adhesive layer 54 can be disposed upon an appointed inwardly facing surface of substrate member 48. The portion of adhesive positioned on factory-bond 50 can be employed to assemble tape fastener 44 onto diaper 20 during the manufacture of the diaper. The portion of adhesive layer 54 located on user-bond region 52 can be employed to secure the diaper onto an infant. The particular adhesive parameters of adhesive layer 54 can be selected and tailored to meet desired adhesive properties, such as adhesive shear strength and adhesive peel strength.

Suitable materials for constructing fasteners 44, such as sheet materials for constructing substrate member 48 and adhesive materials for constructing layer 54, are available from various manufacturers, such as 3M Company, a business having a Disposable Products Division with offices in the 3M Center, St. Paul, Minnesota; and Avery International, a business having a Specialty Tape Division with offices in Painesville, Ohio.

The illustrated embodiment of the tape fastening system includes a release tape member 74 for releasably holding user-bond region 52 of the tape fastener in a storage position which protects the user-bond region of primary adhesive layer 54 against contamination or premature adhesion against other portions of diaper 20. In the illustrated embodiment, release tape 74 is positioned in a superposed, adjacent relation with substrate member 48, and is attached to an interior surface of diaper 20. The representatively shown embodiment of release tape 74 includes an anchor surface 76 and an opposite release surface 78. Anchor surface 76 has disposed thereon a suitable anchor adhesive layer, and release surface 78 has disposed thereon a selected layer of an operable release coating, such as a coating composed of cured (cross-linked) poly dimethyl siloxane (PDMS). Suitable release tapes are commercially available from vendors such as 3M Company and Avery International. For example, suitable release tape materials include the FT-4430 material available from Avery International. The release tape material includes a release surface against which the adhesive bearing surface of the fastening tab can be stored and protected from contamination. The fastening adhesive, however, readily separates from the release surface when desired.

In a particular embodiment of the invention, a terminal end portion of release tape 74 may optionally overlap and adhesively bond to an intermediate section of substrate member 48 along a bond region which traverses across the length of the substrate member. The resultant interconnection between substrate member 48 and release tape 74 provides for a Y-bond which can strengthen the assembly and attachment of tape fastener 44 to the section of diaper 20 that is clamped between release tape 74 and factory-bond region 50 of tape substrate member 48. In other aspects of the invention, release tape 74 can be constructed and configured to provide for stress beam section 98.

The user-bond region of tape substrate member 48 has a distal end section 108 which is appointed for grasping by the user to suitably position and adhere the user-bond region of tape fastener 44 to an appointed tape securement zone of the article. In the illustrated embodiment, for example, the user will typically grasp end section 108 to adhere the tape fastener against landing zone patch 46. Distal end section 108 can be constructed to be non-adhering and non-securing so that the end section can be more easily found and lifted by the user.

In a particular aspect of the invention, tape fastener 44 can include a separate finger tab member 70 connected to substrate end section 60 along an attachment region. The representatively shown overlapping-type bond may comprise an adhesive bond, sonic bond, thermal bond or the like. The finger tab may be overlapped directly against adhesive layer 54, or may be overlapped against the surface of substrate member 48 which is opposite adhesive layer 54, as desired. Where finger tab 70 connects against adhesive layer 54, substrate 48 may be configured to overlap the complete surface area of the finger tab.

In an optional configuration of the invention, finger tab 70 may be constructed by providing a particular physical or chemical treatment applied to end section 60 of substrate member 48. In the illustrated embodiment, for example, the finger tab can be a layer of release tape material. In alternative configurations, the treatment can be configured to impart desired absorbency and/or tactile characteristics to the gripping region of the resultant finger tab.

In yet other aspects or the Invention, finger tab 70 can be composed of a material which is capable of absorbing selected amounts of contaminants, such as powders, liquids, and creams, which may be carried on the fingers of the user. In one aspect of the invention, the finger tab material, particularly the portion comprising the grasping section of the finger tab, provides for an absorbent capacity (absorbency) value of at least about 8 wt%, with respect to white mineral oil having a Saybolt viscosity of about 80 - 90 at 37.8°C (100°F).

Finger tab 70 may also be composed of a material which provides a tactile complement or contrast when compared to substrate member 48. In a particular aspect of the invention, for example, finger tab 70 can include a material which provides a gripping surface having a coefficient of friction value which is not less than about 0.12. A suitable technique for determining the coefficient of friction value is provided by a KAWABATA, Model KES-FB-4, Surface Characteristics Testing Apparatus, a device produced by KATO TECH Co. Ltd, Kyoto, Japan and available from TEX-MAC, a business having offices located at Charlotte, forth Carolina. The apparatus includes a test method for measuring a coefficient of friction value designated ^{"}MIU".

Finger tab 70 can also be configured to provide a gripping surface having a surface roughness value which is not less than about 2.75 micrometers. A suitable technique for determining the surface roughness value is the aforementioned KAWABATA Surface Characteristics Testing Apparatus. The apparatus includes a test method for measuring a surface roughness value designated "SMD".

With still a further aspect of the invention, finger tab 70 can be configured to provide a visually contrasting appearance when compared to substrate member 48. For example, the finger tab may be colored or may include printed graphics which can help to visually distinguish the finger tab from other portions of diaper 20. In preferred configurations, the peripheral end contour of the finger tab is curved and substantially free of sharp corners which might excessively irritate the skin of the wearer.

Finger tab 70 includes a length dimension and a width dimension, and can be configured to provide a grasping area of at least about 39 mm². Alternatively the grasping area is at least about 128 mm², and optionally is at least about 253 mm². In preferred arrangements, the grasping area provided by finger tab 70 can be within the range of about 150 - 300 mm².

The grasping area of the finger tab in combination with the selected dimensions of the finger tab can help increase the area of the finger tab which makes actual contact with the user's thumb and finger during grasping. As a result, the user can more readily hold the tab and can better avoid contact with the primary adhesive layer 54.

The ease of gripping the finger tab may be increased when the general shape of the finger tab approximately matches the shape of the gripping thumb and finger. With particular configurations of the finger tab, the grasping area provided by the finger tab is at least about 20 mm², more preferably is at least about 64 mm², and more preferably is at least about 127 mm². In preferred arrangements, the grasping area provided by finger tab 70 is within the range of about 37 - 75 mm².

The material of finger tab 70 may be substantially coextensive with the width of substrate end section 60, and may substantially end at the longitudinally terminal edge. Alternatively, finger tab 70 may extend beyond the terminal edge of the tape substrate member.

In further aspects of the invention, the fastening system may incorporate a primary stress beam section 98 and at least another supplemental beam section 99, as representatively shown in Figs. 15 and 16. The supplemental beam section may be substantially coterminous with waistband end section 172 (Fig. 15), or may be spaced away from the terminal edge of the waistband end section by a selected discrete distance (Fig. 16).

In the illustrated configurations a waistband section, such as rear waistband section 40 of the article, has at least one lateral end region 172 to which is attached a side panel 90. Typically, the article has another oppositely located waistband end region which has a similar, mirror-image configuration and construction. End region 172 can include a supplemental stress beam section 99 which extends along the length dimension of the waistband end region and also has a selected width dimension. The construction of stress beam section 99 can incorporate the various structures and configurations described with regard to the primary stress beam section 98.

The illustrated configurations have the length of the supplemental stress beam 99 arranged to be substantially coterminous with the corresponding length dimension of the associated waistband end section 172. Alternatively, however, the length of the supplemental stress beam section can be less than the length dimension of the waistband end section. The length of stress beam 99 is, however, longer than the length dimension of side panel 90. In addition, it can be desireable to approximately center the length of the supplemental stress beam member along the length of the waistband end region.

When employing the supplemental stress beam 99, the supplemental beam Is able to accept the force imparted through side panel 90 and distribute the force over a wider area of the chassis structure of an article, such as diaper 20. This can avoid undesirable stress concentrations that might tear or excessively deform localized areas of the diaper components.

The following EXAMPLES are provided to give a more detailed understanding of the invention. The particular amounts, proportions, compositions and parameters are meant to be exemplary, and are not intended to specifically limit the scope of the invention.

### EXAMPLES 1 - 4: Effect of Rigid Stress Beam

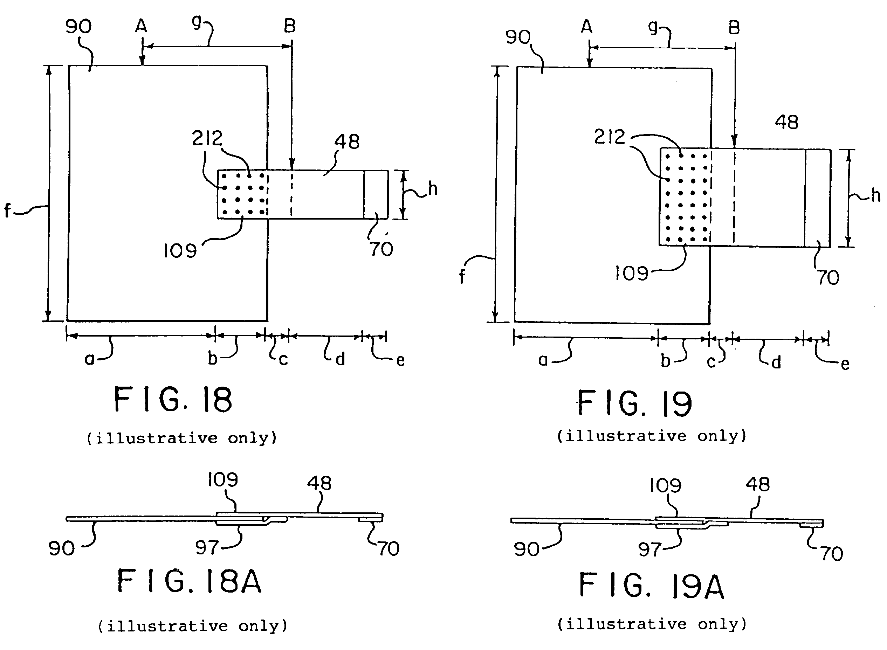

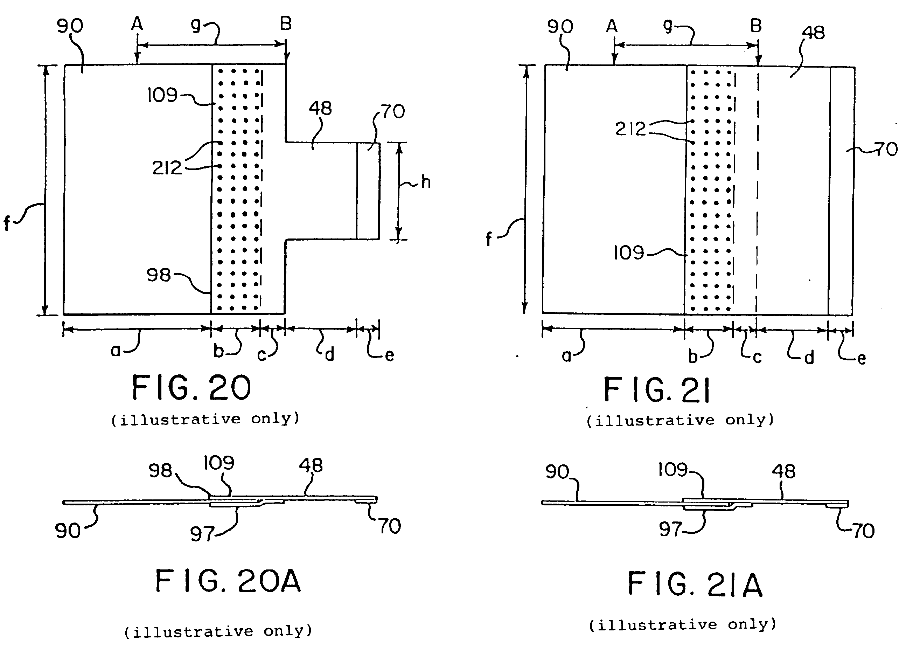

To determine the effect or employing a fastening system having a stress beam section incorporated into a side panel 90 composed of a stretchable elastomeric material, four samples were made with different beam lengths and tape lengths, as illustrated in
Figs. 18 - 21. Code A (Example 1) is representatively shown in
Figs. 18 and 18A; Code B (Example 2) is representatively shown in
Fics. 19 and 19A; Code C (Example 3) is representatively shown in
Figs. 20 and 20A; and Code D (Example 4) is representatively shown in
Figs. 21 and 21A. All Figures 18-21 do not show the inventive concept of the present invention.

In each code, a factory bond laminate 109 included a selected factory bond portion of the tape substrate 48, a selected portion of side panel 90 which overlapped one side the factory bond section of the tape substrate, and a reinforcement member 97 which overlapped an opposite side of the factory bond section of the tape substrate. The three layers were ultrasonically bonded together with the illustrated bonding pattern 212. Tape substrate 48 was composed of 3M KR-3221 attachment tape material, side panel 90 was composed of urethane stretch bonded laminate (SBL) material, and a reinforcement member 97 was composed of 3M KS-0080 release tape material. Finger tab 70 was composed of the release tape material and was adhered to the user bond region of fastener tab substrate 48.

The 3M KR-3221 tape substrate material was about 0.011 cm (about 4.5 mil) thick and was composed of a 0.01 cm (4 mil) thick, transparent polypropylene film coated with about 32 grams per square meter of solvent cured, polystyrene:isoprene block copolymer adhesive. The 3M KS-0080 release tape material was about 0.0089 cm (about 3.5 mil) thick, and was composed of a white polypropylene film. One surface of the film had a release coating composed of poly demethylsiloxane, and an opposed surface of the film had an adhesive layer composed of natural rubber, aliphatic resin and titanium dioxide. The SBL material was about 0.178 cm (about 70 mil) thick, and was composed of a Deerfield X1194, about 0.003 cm (about 1.2 mil) thick urethane film sandwitched between two composite nonwowen fabric webs. Each composite nonwoven web included an outlet nonwoven layer composed of about 13.6 g/m² (0.4 ounce per square yard) polypropylene spunbond, and an inner nonwoven layer composed of 7 gsm, 2 denier, meltblown polypropylene fibers. The complete laminate had a total thickness of about 0.10 cm (about 40 mil). The Gurley stiffness of the SBL material, determined with respect to the lateral width direction, was about 11 mg while the Gurley stiffness of the composite stress beam, determined with respect to the width direction, was about 3920 mg.

In each of Figs. 18 through 21, the indicated dimensions had the following values: "a" = 5.08 cm (2 in); "b" 1.27 cm (0.5 in); "c" = 0.63 5 cm (0.25 in); "d" = 1.9 cm (0.75 in); "e" = 0.635 cm (0.25 in); and "f" = 6.68 cm (2.63 in). With regard to dimension "h"; in Fig. 18, "h" = 1.27 cm (0.5 in); and in Figs. 19 and 20, "h" = 2.54 cm (1 in).

Tensile tests were performed to determine the tensile load-crosshead travel distance curve for each of the four samples. A suitable technique for determining the tensile load-crosshead travel distance curve is a modified version of ASTM Standard Test Method D 882 (Tensile Method for Tensile Properties of Thin Plastic Sheeting). The following modifications are made to the standard procedure:
(1) The rate of separation imparted to the grip members of the testing apparatus is kept at a rate of 50 mm/min for all samples; and (2) The initial separation between grip members is 3.81 cm (1.5 in), which is indicated by the distance "g" between positions ^{"}A^{"} and "B" in Figs. 18 - 21.

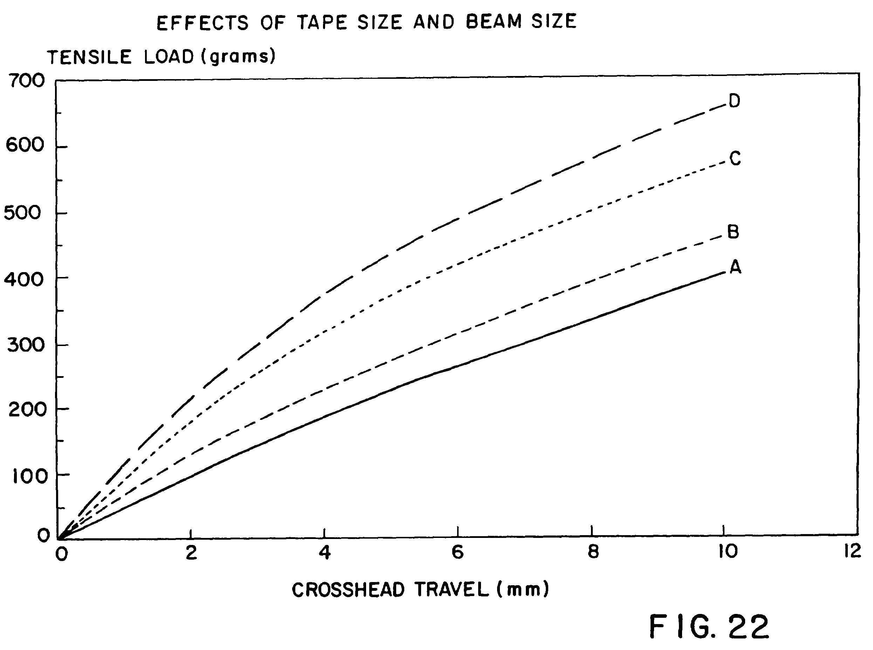

The load-crosshead travel distance curves for these four samples are shown in Fig. 22. Among the four samples, Code A exhibited the lowest and least desireable load-distance curve because it had the shortest factory-bond laminate length and shortest tape length. The effect of the length of the factory-bond laminate 109 can be seen by comparing the curves for Code B and Code C. Both codes had the same tape length of 2.54 cm (1"). However, Code B had a factory-bond laminate length of about 2.54 cm (about 1"), while Code C had a factory-bond laminate length of about 6.68 cm (about 2.63"), which was the same as the panel length. Fig. 22 shows that, at a given crosshead travel distance, the force carried by Code C was higher than the force carried by Code B. This indicates that more of the SBL material in the side panel was being effectively utilized and stretched due to the presence of the longer factory-bond laminate length and the stress beam configuration employed in Code C. The stress beam configuration advantageously spread the applied force over a greater area of the SBL material. More of the SBL material was able to react against the applied load, and at the given crosshead travel distance, a larger total load was being effectively carried by the SBL material.

The effect of the stress beam configuration can also be seen by comparing Code C and Code D. In Code D, the whole SBL panel is stretched since its tape length and factory-bond laminate length are both the same as the panel length. In Code C, force is applied to the tape with a length of 2.54cm (1") which is only 38% of the tape length in Code D. However, at a given crosshead travel distance, the force carried by Code C is about 80% of the force carried by Code D. This indicates how the presence of the relatively rigid stress beam configuration allows the use of a smaller 2.54 cm (1") tape while still effectively utilizing the stretchability and load carrying ability of a greater area of the material in the side panel section.

In these examples, the rigidity (e.g. Gurley stiffness) of the factory-bond laminate was the same for all four samples. If the rigidity of the factory-bond laminate is increased, however, it is expected that, at a given tape length and factory-bond laminate length, a greater area of SBL material will be stretched. As a result, the total force carried by the SBL material at a given crosshead travel distance will be higher.

### EXAMPLES 5 - 7: Effect of Tape Geometry on Resistance to Shear Failure

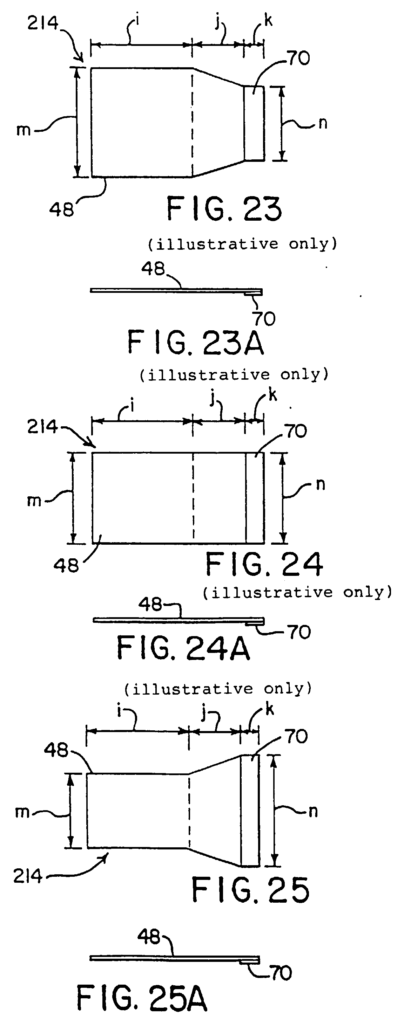

Experiments were conducted to evaluate the effect of the attachment tape geometry on the resistance to shear failure between the fastening tape and the substrate to which the tape is fastened. Three tape samples had the different geometries representatively shown in Fig. 23 (Sample 5), Fig. 24 (Sample 6) and Fig. 25 (Sample 7). Fig. 23, 24 as well as Fig. 26 below do not show the inventive concept according to the present invention, In each sample, the total length of the attachment tape was 5.33 cm (2.1"). In Figs. 23 through 25, the indicated dimensions had the following values: "i" = 3.56 cm (1.4 in); "j" = 1,78 cm (0.70 in); and "k" 0.64 cm (0.25 in). In Fig. 23, "m" = 3.81 cm (1.5 in) and "n" = 2.54 cm (1 in). In Fig. 24, both "m" and "n" = 3.175 cm (1.25 in). In Fig. 25, "m" = 2.54cm (1 in) and "n" = 3.81 cm (1.5 in).

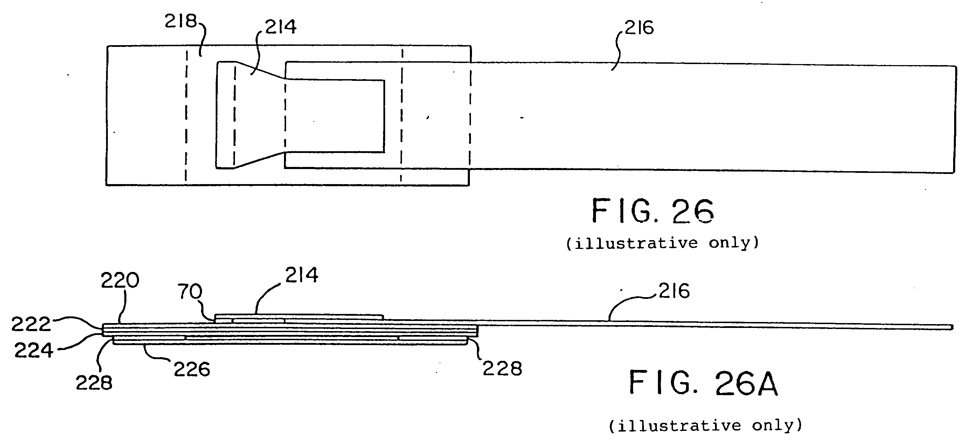

With reference to Figs. 26 and 26A, the first 1.78cm (0.7") section of the attachment tape test sample 214 was adhered to a testing substrate 218 while the 3.56 cm (1.4" of the tape test sample was adhered to a leading strip 216. All three of the sample tapes had the same adhesive and the same adhesive test area of 5.64 cm² (0.875 in²). The testing substrate 218 was a laminate which included a contacting layer 220 composed of release tape (3M KS0080) and diaper outer cover material. The outer cover was a composite composed of 25.4 µm (one mil) thick polyethylene (PE) film 222 and a cellulosic tissue sheet 224. The testing substrate was fixedly secured to the top and bottom part of a rigid, substantially non-deforming stainless steel panel 226 by a pair of spaced apart, 2.54 cm (1") wide, double-sided adhesive tape sections 228, and the stainless steel panel had a size of 5.03 cm x 12.7 cm (2" x 5"). The leading strip 216 measured 3.81 cm (1.5") wide by 22.9 cm (9") long, and can be any suitable kind of material, such as a kraft wrapping paper, which does not break or substantially stretch during the test. The test sample tab was pressed down with a standard 2:05 kg (4.5 lb) mechanical roller (available from Chemsultants International, a company having offices in Mentor, Ohio) by rolling the roller across the test tab once in each direction. The shear test was then conducted immediately thereafter. When placing the test specimen in the tensile tester, the jaws of the tester were initially set 12.7 cm (5") apart. 2.54 cm (one inch) of the bottom of the steel test panel was secured in the stationary jaw with the unsecured leading strip extending past the position of the stationary jaw. The leading strip was clamped in the moving jaw. The moving jaw traveled in the direction away from the stationary jaw at a speed of 100 mm/min until the tape failed in a shear mode. The total energy required to break the adhesive bond in the shear mode was the area under the load-crosshead travel distance curve plotted from the load and distance data generated during the test. A suitable method for calculating the area under the curve is described in ASTM Standard Test Method D 882 (Tensile Method for Tensile Properties of Thin Plastic Sheeting), October 1983.

The Table below shows the results of the testing to determine the total energy required to break the adhesive bond in shear for the three tape geometries.

| Tape Geometry | Energy Required | |
|---|---|---|
| | (inch-pounds) | (N-m) |
| Sample 5 | 0.966 | 0.109 |
| Sample 6 | 1.164 | 0.132 |
| Sample 7 | 1.332 | 0.151 |

The results show that the resistance to shear failure depends on the tape geometry. The tape geometry of the invention, as represented by Sample 7, significantly increased the energy required to cause a shear failure of the fastening bond.

### EXAMPLES 8 -13

Gurley stiffness values were measured for the following samples. With regard to the listed "Sample Size" data, the first measurement corresponds to the "width" and the second measurement corresponds to the actual "length" of the test sample, as such width and length dimensions are determined in accordance with the Gurley stiffness testing procedure.

| Sample No. | Material | Sample Size cm (in) | Gurley Stiffness (mg) |
|---|---|---|---|
| 8 | 25.4µm (1 mil) PE (polyethylene) | 5.08 cm x 2.54 cm (2" x 1") | 0.826 |
| 9 | SBL (urethane film) | 5.08 cm x 2.54cm (2" x 1") | 11.2 |
| 10 | 101.6 µm (4 mil) PP (polypropylene) | 5.08 cm x 2.54cm (2" x 1") | 59.8 |
| 11 | SBL and 101.6 µm (4 mil) PP (composite laminate) | 5.08 cm x 2.54 cm (2" x 1") | 620 |
| 12 | Stress beam section as constructed for Example 3 | 2.54 cm x 3.81 cm (1" x 1.5") | 3920 |
| 13 | Cardboard | 1.27cm x 11.43cm (0.5" x 4.5") | 84760 |

## Claims

1. A fastening system comprising a fastening tab (44) and a separate stress beam reinforcement section (98), said fastening tab (44) comprising a tab substrate (48) having a factory bond section (50), a user bond section (52), and a seam section (69) which is located between said factory bond (50) and user bond section (52), said stress beam reinforcement section (98) being assembled to overlap on a surface of said factory bond section (50) of said tab substrate (48), said user bond section (52) having a length dimension (62), in a longitudinal direction (86) of an article to be fastened, which is larger than a length dimension of said seam section (69).

2. The fastening system according to claim 1, wherein said tab substrate (48) has an adhesive (54) disposed on an exposed surface thereof.

3. The fastening system according to claim 1, wherein said tab substrate (48) has a cooperating component of a mechanical fastening system connected to an exposed surface of said tab substrate (48).

4. The fastening system according to claim 3, wherein said tab substrate (48) includes a cooperating component of a hook-and-loop-type fastening system connected to said exposed surface of said tab substrate (48).

5. An article comprising the fastening system according to any of the preceding claims, said article further comprising a front waistband portion (38), a rear waistband portion (40), an intermediate portion (42) interconnecting said front and rear waistband portions, and a backsheet layer (22), said fastening system being connected to opposed lateral ends of at least one waistband portion (38, 40) of said backsheet layer (22) for securing said article on a wearer.

6. The article according to claim 5 further comprising a pair of side panels (90), each of which extends laterally at opposed lateral ends of the at least one waistband portion of said backsheet layer (22), each of said side panels including a terminal free end region (92) which has a predetermined length dimension (94) thereof;
said stress beam reinforcement section (98) of said fastening system being connected to each of said side panels (90) along said free end region (92), said stress beam reinforcement section (98) providing for a Gurley stiffness value of at least about 20 mg and having a length dimension which is at least about 33% of said length (94) of the free-end region of said side panel;
said fastening tab (44) being connected to each of said stress beam reinforcement sections (98) arranged to extend laterally from each of said side panels (90) for securing said article during use thereof, said fastening tab (44) having a base length (58) which is not more than about 90% of said length (102) of said stress beam reinforcement section (98).

7. The article according to claim 5 further comprising a pair of side panels (90), each of which extends laterally at opposed lateral ends of the at least one waistband portion of said backsheet layer (22), each of said side panels including a terminal free end region (92) which has a predetermined length dimension (94) thereof;
said stress beam reinforcement section (98) being connected to each of said side panels (90) along said free end region (92), said stress beam reinforcement section (98) having a length dimension which is at least about 33% of said length (94) of the free-end region of said side panel;
said fastening tab (44) being arranged to extend laterally from each of said side panels (90) for securing said article during use thereof, said fastening tab (44) having a base length (58) which is not more than about 99% of said length (102) of said stress beam reinforcement section (98), said fastening tab (44) leaving opposed ends of said stress beam reinforcement sections (98) substantially unsecured during said securing of the article on the wearer.

8. The article according to claim 7, wherein said side panels (90) are composed of a material having a Gurley stiffness value of not more than about 10000 mg.

9. The article according to claim 8, wherein said stress beam reinforcement section (98) has a Gurley stiffness value of at least about 100 mg.

10. The article according to claim 9, wherein said stress beam reinforcement section (98) provides for a Gurley stiffness value of not more than about 50000 mg.

11. The article according to claim 10, wherein each stress beam reinforcement section (98) and its connected side panel (90) have a stiffness ratio of at least about 5:1.

12. The article according to claim 6, wherein said fastening tabs (44) have a stiffness which is not more than said stiffness of said stress beam reinforcement section (98).

13. The article according to claim 12, wherein said fastening tabs (44) have a Gurley stiffness of at least about 5 mg.

14. The article according to claim 13, wherein said fastening tabs (44) have a Gurley stiffness value of not more than about 500 mg.

15. The article according to claim 6, wherein said fastening tabs (44) have a seam section (69) with a length of not less than about 0.5 cm.

16. The article according to claim 15, wherein said fastening tabs (44) have a seam section (69) with a length of not more than about 12.5 cm.

17. The article according to claim 6, wherein said side panels (90) are elastically stretchable along a lateral cross-direction of said article.

18. The article according to claim 17, wherein said side panels (90) are composed of an elastomeric nonwoven fabric.

19. The article according to claim 6, further comprising a liquid permeable topsheet (24) superposed in facing relation with said backsheet (22); and an absorbent body (26) sandwiched between said topsheet (24) and backsheet (22).

## Patentansprüche

1. Befestigungssystem, umfassend eine Befestigungslasche (44) und einen getrennten Beanspruchungsbalkenverstärkungsabschnitt (98), wobei die Befestigungslasche (44) ein Laschensubstrat (48) umfaßt, welches einen Fabrikationsverbindungsabschnitt (50), einen Benutzerverbindungsabschnitt (52) und einen Nahtabschnitt (69) aufweist, welcher zwischen dem Fabrikationsverbindungsabschnitt (50) und dem Benutzerverbindungsabschnitt (52) angeordnet ist, wobei der Beanspruchungsbalkenverstärkungsabschnitt (98) angebracht ist, um sich mit einer Oberfläche des Fabrikationsverbindungsabschnittes (50) des Laschensubstrats (48) zu überlappen, wobei der Benutzerverbindungsabschnitt (52) ein Längenmaß (62) in einer Längsrichtung (86) eines zu befestigenden Artikels aufweist, welches größer als ein Längenmaß des Nahtabschnittes (69) ist.

2. Befestigungssystem nach Anspruch 1, wobei das Laschensubstrat (48) einen Klebstoff (54) aufweist, welcher auf einer freiliegenden Oberfläche davon angeordnet ist.

3. Befestigungssystem nach Anspruch 1, wobei das Laschensubstrat (48) eine zusammenwirkende Komponente eines mechanischen Befestigungssystems aufweist, welche mit einer freiliegenden Oberfläche des Laschensubstrats (48) verbunden ist.

4. Befestigungssystem nach Anspruch 3, wobei das Laschensubstrat (48) eine zusammenwirkende Komponente eines Haken/Schlaufen-Befestigungssystems aufweist, welche mit einer freiliegenden Oberfläche des Laschensubstrats (48) verbunden ist.

5. Artikel, umfassend das Befestigungssystem nach einem der vorhergehenden Ansprüche, wobei der Artikel des weiteren einen vorderen Bundabschnitt (38), einen hinteren Bundabschnitt (40), einen Zwischenabschnitt (42), welcher den vorderen und den hinteren Bundabschnitt miteinander verbindet, und eine Grundlage (22) umfaßt, wobei das Befestigungssystem mit entgegengesetzten seitlichen Enden von mindestens einem Taillenbundabschnitt (38, 40) der Grundlage (22) verbunden ist, um den Artikel an einem Träger zu befestigen.

6. Artikel nach Anspruch 5, des weiteren umfassend ein Paar Seitenstreifen (90), wovon sich jeder seitlich an entgegengesetzten seitlichen Enden des mindestens einen Bundabschnittes der Grundlage (22) erstreckt, wobei jeder der Seitenstreifen einen abschließenden freien Endbereich (92) umfaßt, welcher ein vorgegebenes Längenmaß (94) davon aufweist;
wobei der Beanspruchungsbalkenverstärkungsabschnitt (98) des Befestigungssystems mit jedem der Seitenstreifen (90) entlang dem freien Endbereich (92) verbunden ist, wobei der Beanspruchungsbalkenverstärkungsabschnitt (98) einen Gurley-Steifigkeitswert von mindestens 20 mg vorsieht und ein Längenmaß aufweist, welches mindestens ungefähr 33 % der Länge (94) des Freiendbereiches des Seitenpaneels beträgt;
wobei die Befestigungslasche (44) mit jedem der Beanspruchungsbalkenverstärkungsabschnitte (98) verbunden ist, welche angeordnet sind, um sich seitlich von jedem der Seitenstreifen (90) zu erstrecken, um den Artikel während dessen Gebrauch zu befestigen, wobei die Befestigungslasche (44) an der Basis eine Länge (58) aufweist, welche höchstens ungefähr 90 % der Länge (102) des Beanspruchungsbalkenverstärkungsabschnittes (98) beträgt.

7. Artikel nach Anspruch 5, des weiteren umfassend ein Paar Seitenstreifen (90), wovon sich jeder seitlich an entgegengesetzten seitlichen Enden des mindestens einen Bundabschnittes der Grundlage (22) erstreckt, wobei jeder der Seitenstreifen einen abschließenden freien Endbereich (92) umfaßt, welcher ein vorgegebenes Längenmaß (94) davon aufweist;
wobei der Beanspruchungsbalkenverstärkungsabschnitt (98) mit jedem der Seitenstreifen (90) entlang dem freien Endbereich (92) verbunden ist, wobei der Beanspruchungsbalkenverstärkungsabschnitt (98) ein Längenmaß aufweist, welches mindestens ungefähr 33 % der Länge (94) des Freiendbereiches des Seitenpaneels beträgt;
wobei die Befestigungslasche (44) angeordnet ist, um sich seitlich von jedem der Seitenstreifen (90) zu erstrecken, um den Artikel während dessen Gebrauch zu befestigen, wobei die Befestigungslasche (44) an der Basis eine Länge (58) aufweist, welche höchstens ungefähr 99 % der Länge (102) des Beanspruchungsbalkenverstärkungsabschnittes (98) beträgt, wobei die Befestigungslasche (44) entgegengesetzte Enden der Beanspruchungsbalkenverstärkungsabschnitte (98) während des Befestigens des Artikels am Träger im wesentlichen unbefestigt läßt.

8. Artikel nach Anspruch 7, wobei die Seitenstreifen (90) aus einem Material bestehen, welches einen Gurley-Steifigkeitswert von höchstens ungefähr 10000 mg aufweist.

9. Artikel nach Anspruch 8, wobei der Beanspruchungsbalkenverstärkungsabschnitt (98) einen Gurley-Steifigkeitswert von mindestens ungefähr 100 mg aufweist.

10. Artikel nach Anspruch 9, wobei der Beanspruchungsbalkenverstärkungsabschnitt (98) einen Gurley-Steifigkeitswert von höchstens ungefähr 50000 mg vorsieht.

11. Artikel nach Anspruch 10, wobei jeder Beanspruchungsbalkenverstärkungsabschnitt (98) und sein angeschlossener Seitenstreifen (90) ein Steifigkeitsverhältnis von mindestens ungefähr 5:1 aufweisen.

12. Artikel nach Anspruch 6, wobei die Befestigungslaschen (44) eine Steifigkeit aufweisen, welche die Steifigkeit des Beanspruchungsbalkenverstärkungsabschnittes (98) nicht übersteigt.

13. Artikel nach Anspruch 12, wobei die Befestigungslaschen (44) eine Gurley-Steifigkeit von mindestens ungefähr 5 mg aufweisen.

14. Artikel nach Anspruch 13, wobei die Befestigungslaschen (44) einen Gurley-Steifigkeitswert von höchstens ungefähr 500 mg aufweisen.

15. Artikel nach Anspruch 6, wobei die Befestigungslaschen (44) einen Nahtabschnitt (69) mit einer Länge von mindestens ungefähr 0,5 cm aufweisen.

16. Artikel nach Anspruch 15, wobei die Befestigungslaschen (44) einen Nahtabschnitt (69) mit einer Länge von höchstens ungefähr 12,5 cm aufweisen.

17. Artikel nach Anspruch 6, wobei die Seitenstreifen (90) entlang einer seitlichen Querrichtung des Artikels elastisch dehnbar sind.

18. Artikel nach Anspruch 17, wobei die Seitenstreifen (90) aus einem Elastomer-Vliesstoff bestehen.

19. Artikel nach Anspruch 6, des weiteren umfassend eine flüssigkeitsdurchlässige Decklage (24), welche in einer gegenüberliegenden Beziehung über die Grundlage (22) gelegt ist; und einen saugfähigen Körper (26), welcher zwischen der Decklage (24) und der Grundlage (22) eingeklemmt ist.

## Revendications

1. Système d'attache comprenant une patte d'attache (44) et une zone de poutre d'efforts séparée (98), ladite patte d'attache (44) comprenant un substrat de ruban (48) ayant une zone de liaison à la fabrication (50), une zone de liaison utilisateur (52) et une zone de réunion (69) qui est disposée entre ladite zone de liaison à la fabrication (50) et ladite zone de liaison utilisateur (52), ladite zone de poutre d'efforts (98) étant assemblée pour recouvrir une surface de ladite zone de liaison à la fabrication (50) audit substrat de ruban (48), ladite zone de liaison utilisateur (52) ayant une dimension longitudinale (62), dans une direction longitudinale (86) d'un article à fixer, qui est supérieure à la dimension longitudinale de ladite zone de réunion (69).

2. Système d'attache selon la revendication 1, dans lequel ledit substrat de ruban (48) comporte un adhésif (54) placé sur une surface exposée dudit substrat.

3. Système d'attache selon la revendication 1, dans lequel ledit substrat de ruban (48) comprend un composant coopérant d'un système de fixation mécanique qui est connecté à une surface exposée dudit substrat de ruban (48).

4. Système d'attache selon la revendication 3, dans lequel ledit substrat de ruban (48) comprend un composant coopérant d'un système d'attache du type à crochets et boucles connecté à une surface exposée dudit substrat de ruban (48).

5. Article comprenant un système d'attache selon l'une quelconque des revendications précédentes, ledit article comprenant en outre une portion de ceinture avant (38), une portion de ceinture arrière (40), une portion intermédiaire (42) interconnectant lesdites portions de ceinture avant et arrière, et une couche de feuille support (22), ledit système d'attache étant connecté aux extrémités latérales opposées d'au moins une portion de ceinture (38,40) de ladite couche de feuille support (22) pour fixer ledit article sur un porteur.

6. Article salon la revendication 5, comprenant en outre une paire de panneaux latéraux (90), dont chacun s'étend latéralement aux extrémités latérales opposées d'au moins une portion de ceinture de ladite couche de feuille support (22), chacun desdits panneaux latéraux comportant une région d'extrémité libre (92) terminale qui a une dimension longitudinale (94) prédéterminée ;
ladite zone de poutre d'efforts (98) dudit système d'attache étant connectée à chacun desdits panneaux latéraux (90) le long de ladite région d'extrémité libre (92), ladite zone de poutre d'efforts (98) offrant une valeur de rigidité Gurley d'au moins environ 20 mg et ayant une dimension longitudinale qui est d'au moine environ 33 % de ladite longueur (94) de la région d'extrémité libre dudit panneau latéral ;
ladite patte d'attache (44) étant connectée à chacune desdites zones de poutre d'efforts (98) et disposée pour s'étendre latéralement à partir de chacun desdits panneaux latéraux (90) pour fixer ledit article pendant son utilisation, ladite patte d'attache (44) ayant une longueur de base (58) qui n'excède pas environ 90 % de ladite longueur (102) de ladite zone de poutre d'efforts (98).

7. Article selon la revendication 5, comprenant an outre une paire de panneaux latéraux (90), dont chacun s'étend latéralement aux extrémités latérales opposées d'au moins une portion de ceinture de ladite couche de feuille support (22), chacun desdits panneaux latéraux comportant une région d'extrémité libre (92) terminale qui a une dimension longitudinale (94) prédéterminée ;
ladite zone de poutre d'efforts (98) étant connectée à chacun desdits panneaux latéraux (90) le long de ladite région d'extrémité libre (92), ladite zone de poutre d'efforts (98) ayant une dimension longitudinale qui est d'au moins environ 33 % de ladite longueur (94) de la région d'extrémité libre dudit panneau latéral ;
ladite patte d'attache (44) étant disposée pour s'étendre latéralement à partir de chacun desdits panneaux latéraux (90) pour fixer ledit article pendant son utilisation, ladite patte d'attacha (44) ayant une longueur de base (58) qui n'excède pas environ 99 % de ladite longueur (102) de ladite zone de poutre d'efforts (98), ladite patte d'attache (44) laissant les extrémités opposées desdites zones de poutre d'efforts (98) sensiblement non attachées pendant la fixation de l'article sur le porteur.

8. Article selon la revendication 7, dans lequel lesdits panneaux latéraux (90) sont constitués d'un matériau ayant une valeur de rigidité Gurley qui n'excède pas environ 10 000 mg.

9. Article selon la revendication 8, dans lequel ladite zone de poutre d'efforts (98) a une valeur de rigidité Gurley d'au moins environ 100 mg.

10. Article selon la revendication 9, dans lequel ladite zone de poutre d'efforts (98) offre une valeur de rigidité Gurley qui n'excède pas environ 50 000 mg

11. Article selon la revendication 10, dans lequel chaque zone de poutre d'efforts (98) et son panneau latéral connecté (90) ont un rapport de rigidité d'au moins environ 5:1.

12. Article selon la revendication 6, dans lequel lesdites pattes d'attache (44) ont une rigidité qui n'excède pas la rigidité de ladite zone de poutre d'efforts (98).

13. Article selon la revendication 12, dans lequel lesdites pattes d'attache (44) ont une valeur de rigidité Gurley d'au moins environ 5 mg.

14. Article selon la revendication 13, dans lequel lesdites pattes d'attache (44) ont une valeur de rigidité Gurley qui n'excède pas environ 500 mg.

15. Article selon la revendication 6, dans lequel lesdites pattes d'attache (44) ont une zone de réunion (69) ayant une longueur qui n'est pas inférieure à environ 0,5 cm.

16. Article selon la revendication 15, dans lequel lesdites pattes d'attache (44) ont une zone de réunion (69) ayant une longueur qui n'excède pas environ 12,5 cm.

17. Article selon la revendication 6, dans lequel lesdits panneaux latéraux (90) sont extensibles élastiquement selon une direction transversale latérale dudit article.

18. Article selon la revendication 17, dans lequel lesdits panneaux latéraux (90) sont constitués d'une étoffe non tissée élastomère.

19. Article selon la revendication 6, comprenant en outre une feuille supérieure (24) perméable aux liquides en relation de vis-à-vis avec ladite couche de feuille support (22) ; et un corps absorbant (26) pris an sandwich entre ladite feuille supérieure (24) et ladite couche de feuille support (22).
